# EUROPEAN PATENT APPLICATION

(11) **EP 4 710 972 A2**
(43) Date of publication of application: **18.03.2026**
(21) Application number: 26155204.6
(22) Date of filing: 18.01.2023
(51) Int. Cl.: A61M 11/00

(54) **TREPROSTINIL FOR THE TREATMENT OF PULMONARY HYPERTENSION**

(30) Priority: 21.01.2022 EP 22152809
(62) Divisional of application: 23701847.8
(71) Applicant: InvoX Belgium NV, 3590 Diepenbeek (BE)
(72) Inventor: D'OREY MARCHAND SEQUEIRA LOPES BEIRÃO BELO, Isabel, 2780-307 Oeiras (PT); RAWERT, Jürgen, 50933 Köln (DE)
(74) Representative: Brand Murray Fuller LLP

(57) **Abstract**

The invention provides Treprostinil or a pharmaceutically acceptable salt or derivative thereof for use in a method of treatment of pulmonary hypertension in a subject being diagnosed with pulmonary hypertension or being at risk of developing pulmonary hypertension wherein the treatment comprises the steps of
i) administering to said subject a first liquid composition comprising Treprostinil or a pharmaceutically acceptable salt or derivative thereof in a first concentration in aerosolized form for a first treatment period, and
ii) administering to said subject a second liquid composition comprising Treprostinil or a pharmaceutically acceptable salt or derivative thereof in a second concentration in aerosolized form for a second treatment period following the first treatment period;

wherein the second concentration of Treprostinil or a pharmaceutically acceptable salt or derivative thereof in the second liquid composition is higher than the first concentration of Treprostinil or a pharmaceutically acceptable salt or derivative thereof in the first liquid composition, and
wherein the first and the second liquid compositions are aerosolized and administered to the subject using a soft mist inhaler.

## Description

The present invention relates to a method, compositions and kits for the inhalative administration of Treprostinil or a pharmaceutically acceptable salt or derivative thereof. Furthermore, the present invention relates to a pharmaceutical product comprising Treprostinil or a pharmaceutically acceptable salt or derivative thereof for use in the treatment of pulmonary hypertension as well as to a kit comprising solutions of Treprostinil having different concentrations.

### BACKGROUND OF THE INVENTION

Pulmonary hypertension is a medical condition of increased blood pressure within the blood vessels of the lungs. Symptoms include shortness of breath, fainting, tiredness, chest pain, swelling of the legs and an increased frequency of the heartbeat.

According to a WHO classification, there are five groups of pulmonary hypertension wherein such groups may be divided into further subgroups. For example, WHO Group I refers to pulmonary arterial hypertension (PAH) whereas WHO Group III refers to pulmonary hypertension due to lung disease (such as chronic obstructive pulmonary disease, COPD) or chronic hypoxia. As a further example, WHO Group IV refers to chronic arterial obstruction.

Depending on the specific group or type of pulmonary hypertension diagnosed in a patient there are several surgical and pharmaceutical therapeutic approaches available, the latter including the administration of vasoactive substances such as endothelin receptor antagonists, phosphodiesterase type 5 inhibitors, activators of soluble guanylate cyclase and prostaglandins or prostanoid analogues. An example for a stable prostanoid analogue which has been used in the treatment of pulmonary hypertension is Treprostinil which can be administered by intravenous or subcutaneous infusion. While the administration of Treprostinil by intravenous infusing might be associated with an increased risk of infections and sepsis, the administration of Treprostinil by subcutaneous infusion has been reported to be associated with considerable pain. Accordingly, as an alternative, formulations of Treprostinil for inhalative administration have been developed.

US 2020/0171044 A1discloses a method of treating pulmonary hypertension comprising administering by inhalation to a human suffering from pulmonary hypertension a therapeutically effective single event dose of a formulation comprising Treprostinil or a pharmaceutically acceptable salt thereof with a specific inhalation device at a dose of 15 to 90 µg in one to three breaths. The formulations administered have a concentration of Treprostinil of 1 mg/mL or 2 mg/mL, respectively.

R. Voswinkel et al. Disclose in Pulmonary Pharmacology & Therapeutics 22 (2009), p, 50 to 56 the administration of doses of 30, 45 and 60 µg Treprostinil in the form of liquid formulations with a concentration of 1 mg/mL and 2 mg/mL in two or three breaths using a specific inhalation device.

K. S. Parikh et al. report in J. Cardiovasc. Pharmacol., Vol. 67, Number 4, p. 322 to 325 a complex titration algorithm reaching a maximum of initially three breaths, followed by 6 breaths and finally up to 12 breaths comprising 72 µg of Treprostinil four times daily whereas with each breath the fixed amount of 6 µg of Treprostinil is administered.

A pharmaceutical product for inhalation comprising Treprostinil in the form of a solid dry powder (TYVASO DPI^{™}, United Therapeutics Corp.) is approved in the USA. The administration of dry powders, however, has several disadvantages comprising, i. a., a complicated handling due to different dry powder formulations to be provided in different containers, specific storage conditions and short storage times of the dry powder formulations as well as significant amounts of waste materials.

For the only commercially available pharmaceutical product for inhalation comprising Treprostinil (TYVASO^{®}, United Therapeutics Corp.) in liquid form, likewise, a complicated dosage scheme has been developed in which the drug product is dosed in four separate, equally spaced treatment sessions per day. In an initial phase of therapy, the drug product is administered by three breaths of the drug product four times a day. This dosage is increased by an additional three breaths in biweekly intervals to a target dose of nine breaths per treatment session to be applied four times daily.

These complicated dosage regimens inherently bear the risk of over- or underdosing due to human error as the exact number of continuously increasing inhalation breaths has to be counted and documented or the correct containers have to be identified which poses a significant burden on the patient, especially when seriously ill and when having significant functional limitations, as well as on medical staff, if necessary. Furthermore, due to the frequent application by multiple daily inhalations comprising up to 12 inhalation breaths, these protocols and dosage schemes cause a significant inhalation time which may lead to poor patient compliance and to patient treatment withdrawal. In addition, multiple consecutive inhalation manoeuvres may lead to secondary adverse effects such as coughing, throat soreness and others.

It is thus an object of the present invention to provide for a method for the inhalative administration of Treprostinil that allows for the implementation of a simplified administration scheme, if possible during all phases of the treatment, and, accordingly, reduces the risk of over- or under-dosing at reduced overall daily inhalation time and that, accordingly, reduces the burden to the patient in need of such treatment when implementing the required inhalation scheme. Further objects of the invention will be clear on the basis of the following description of the invention, examples and claims.

### SUMMARY OF THE INVENTION

In a first aspect, the invention relates to a method for the inhalative administration of Treprostinil or a pharmaceutically acceptable salt or derivative thereof to a subject in need of such administration, the method comprising the steps of
i) administering to said subject a first liquid composition comprising Treprostinil or a pharmaceutically acceptable salt or derivative thereof in a first concentration in aerosolized form for a first treatment period; and
ii) administering to said subject a second liquid composition comprising Treprostinil or a pharmaceutically acceptable salt or derivative thereof in a second concentration in aerosolized form for a second treatment period following the first treatment period;

wherein the second concentration of Treprostinil or a pharmaceutically acceptable salt or derivative thereof in the second liquid composition is higher than the first concentration of Treprostinil or a pharmaceutically acceptable salt or derivative thereof in the first liquid composition; and
wherein the first and the second liquid compositions are aerosolized and administered to the subject using a soft mist inhaler.

In a further aspect, the present invention provides a pharmaceutical product comprising Treprostinil or a pharmaceutically acceptable salt or derivative thereof for use in the treatment of pulmonary hypertension in a subject being diagnosed with pulmonary hypertension or for use in the prevention of pulmonary hypertension in a subject being at risk of developing pulmonary hypertension, the treatment or prevention comprising the steps of
i) administering to said subject a first liquid composition comprising Treprostinil or a pharmaceutically acceptable salt or derivative thereof in a first concentration in aerosolized form for a first treatment period; and
ii) administering to said subject a second liquid composition comprising Treprostinil or a pharmaceutically acceptable salt or derivative thereof in a second concentration in aerosolized form for a second treatment period following the first treatment period;

wherein the second concentration of Treprostinil or a pharmaceutically acceptable salt or derivative thereof in the second liquid composition is higher than the first concentration of Treprostinil or a pharmaceutically acceptable salt or derivative thereof in the first liquid composition, and
wherein the first and the second liquid compositions are aerosolized and administered to the subject using a soft mist inhaler.

In a third aspect, the invention provides for a kit comprising
a) a first liquid composition comprising Treprostinil or a pharmaceutically acceptable salt or derivative thereof in a first concentration; and
b) a second liquid composition comprising Treprostinil or a pharmaceutically acceptable salt or derivative thereof in a second concentration; and optionally
c) a third liquid composition comprising Treprostinil or a pharmaceutically acceptable salt or derivative thereof in a third concentration; and
d) a soft mist inhaler;
wherein the second concentration of Treprostinil or a pharmaceutically acceptable salt or derivative thereof in the second liquid composition is higher than the first concentration of Treprostinil or a pharmaceutically acceptable salt or derivative thereof in the first liquid composition and wherein the third concentration of Treprostinil or a pharmaceutically acceptable salt or derivative thereof in the optional third liquid composition is higher than the second concentration of Treprostinil or a pharmaceutically acceptable salt or derivative thereof in the second liquid composition.

### BRIEF DESCRIPTION OF THE DRAWINGS

Figure 1 depicts the droplet Size distribution (DSD) curve generated when aerosolizing a 1 mg/mL solution of Treprostinil in water with an impingement-type soft mist inhaler.
Figure 2 shows the DSD curve generated when aerosolizing a 1 mg/mL solution of Treprostinil in water with an impingement-type soft mist inhaler. The presented results are the average DSD across 120 device actuations.
Figure 3 shows the DSD curve generated when aerosolizing a 5mg/mL solution of Treprostinil in water with an impingement-type soft mist inhaler.
Figure 4 depicts the DSD curve generated when aerosolizing a 5mg/mL solution of Treprostinil in water/ethanol (50%/50% (v/v)) with an impingement-type soft mist inhaler.
Figure 5 depicts a cross-sectional view of an impingement-type soft mist inhaler with a container inserted.

### DETAILED DESCRIPTION OF THE INVENTION

The inventors developed a significantly improved and simplified method for the inhalative administration of Treprostinil or a pharmaceutically acceptable salt or derivative thereof using a soft mist inhaler. The novel administration method is based on using a plurality of liquid compositions comprising Treprostinil or a pharmaceutically acceptable salt or derivative thereof with different concentrations which are administered by inhalation with a soft mist inhaler. The new and improved administration method is particularly suitable for the treatment of pulmonary hypertension and allows for an easier dosing and administration protocol while increasing the dosage of Treprostinil or a pharmaceutically acceptable salt or derivative thereof over time, and across the several titration treatment phases. Advantages of the method of the invention comprise, but are not limited to, for example; a) a reduced number of breaths per administration, b) a more convenient and easier to follow application regimen (by maintaining the same posology across all of the treatments phases), and c) an easier transition to the next dosage level (and consequently to the next transition phase). Further advantages of the method of the present invention comprise, but are not limited to improved stability profile (especially when compared to dry powder formulations), advantageous storage requirements and a significantly improved applicability (e.g. independent of the actual bearing or position of the subject).

The term "treatment" as used herein, unless otherwise stated, means administration of the compound or composition to a subject to at least ameliorate, reduce or suppress existing signs or symptoms of the disease, disorder or condition, specifically pulmonary hypertension, experienced by the subject.

The term "prevention" as used herein, unless otherwise stated, means prophylactically administering the compound or composition to a subject who does not exhibit signs or symptoms of a disease, disorder or condition, specifically pulmonary hypertension, but who is expected or anticipated to likely exhibit such signs or symptoms in the absence of prevention. Preventative treatment may at least lessen or partly ameliorate expected symptoms or signs.

The term "aerosolization" as used herein, unless otherwise stated means the conversion of a liquid or liquid composition into droplets of such liquid or liquid composition which are small and light enough to be carried on the air to form an aerosol. Accordingly, the terms "aerosolizing" or "aerosolized" as used herein means the process as well as the result of such process by which the liquid or liquid composition is converted into an aerosol.

The term "effective amount" as used herein refers to the administration of an amount of the relevant compound or composition sufficient to prevent the occurrence of symptoms of the condition being treated, or to bring about a halt in the worsening of symptoms or to treat and alleviate or at least reduce the severity of the symptoms. The effective amount will vary in a manner which would be understood by a person of skill in the art with patient age, sex, weight etc.

The term "event" as used herein means, unless otherwise stated, a treatment session during which one dose of the relevant compound is administered to/inhaled by the subject, whereas a treatment session can comprise one or a plurality of activations of the soft mist inhaler.

The term "dose" as used herein means, unless otherwise stated, the amount of the relevant compound administered to the subject in one event.

The term "treatment period" as used herein, unless otherwise stated, means the duration of a treatment phase in which the subject is receiving a liquid composition comprising Treprostinil or a pharmaceutically acceptable salt or derivative thereof in a specific concentration in aerosolized form.

The term "subject" or "individual" or "patient" as used herein, unless otherwise stated, may refer to any subject, particularly a vertebrate subject, and even more particularly a mammalian subject, for whom therapy is desired. Suitable vertebrate animals include, but are not restricted to, primates, avians, livestock animals (e.g., sheep, cows, horses, donkeys, pigs), laboratory test animals (e.g., rabbits, mice, rats, guinea pigs, hamsters), companion animals (e.g., cats, dogs) and captive wild animals (e.g., foxes, deer, dingoes). A preferred subject, individual or patient is a human, such as a male or female human, especially an adult male or female human having an age of from at least 16 years or of at least 18 years, such as from 18 to about 90 years or from about 30 to about 80 years or from about 40 years to about 75 years.

Treprostinil (2-[[(1R,2R,3aS,9aS)-2-hydroxy-1-[(3S)-3-hydroxyoctyl]-2,3,3a,4,9,9a-hexahydro-1H-cyclopenta[g]naphthalen-5-yl]oxy]acetic acid; CAS Nr. 81846-19-7) has a molecular weight of 390.52 g/mol and a chemical structure as depicted in formula (I, R = H) below:

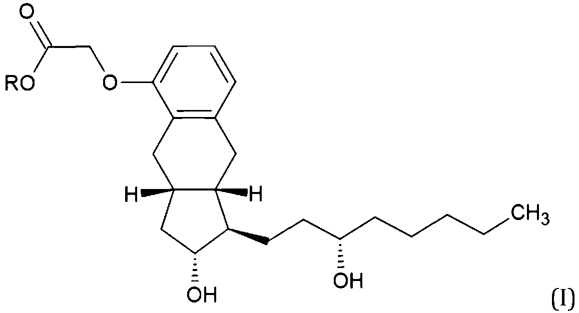

The most important pharmaceutically acceptable salts include, but are not limited to Treprostinil sodium (R = Na) and Treprostinil diolamine (Treprostinil diethanolamine, R = H₂N(C₂H₄OH)₂). Furthermore, pharmaceutically acceptable derivatives of Treprostinil as referred to herein comprise, but are not limited to carboxylic esters of Treprostinil (sometimes also referred to as "ester prodrugs" of Treprostinil of formula (I) with R being an aliphatic hydrocarbon residue having 1 to about 25 carbon atoms or about 6 to about 20 carbon atoms or about 12 to about 20 carbon atoms, preferably a saturated, unbranched hydrocarbon residue), for example Treprostinil Palmitil of formula (I) with R = (CH₂)₁₅CH₃).

According to the first aspect, the invention relates to a method for the inhalative administration of Treprostinil or a pharmaceutically acceptable salt or derivative thereof to a subject in need of such administration, the method comprising the steps of
i) administering to said subject a first liquid composition comprising Treprostinil or a pharmaceutically acceptable salt or derivative thereof in a first concentration in aerosolized form for a first treatment period; and
ii) administering to said subject a second liquid composition comprising Treprostinil or a pharmaceutically acceptable salt or derivative thereof in a second concentration in aerosolized form for a second treatment period following the first treatment period;

wherein the second concentration of Treprostinil or a pharmaceutically acceptable salt or derivative thereof in the second liquid composition is higher than the first concentration of Treprostinil or a pharmaceutically acceptable salt or derivative thereof in the first liquid composition; and
wherein the first and the second liquid compositions are aerosolized and administered to the subject using a soft mist inhaler.

The method of the invention is particularly suitable for the treatment of pulmonary hypertension. Accordingly, in one embodiment, the invention relates to a method as defined above, wherein the subject to be treated is diagnosed with pulmonary hypertension. In particular embodiments, the method as defined above is a method for the treatment of pulmonary hypertension. In some embodiments, the pulmonary hypertension may be an idiopathic or primary hypertension or, in other words a pulmonary hypertension with an unknown cause or a heritable pulmonary hypertension. In other embodiments, the pulmonary hypertension may be secondary pulmonary hypertension resulting from known risk factors or underlying diseases, for example hearth and lung diseases such as chronic hypoxia, chronic obstructive pulmonary disease (COPD), interstitial lung disease, connective tissue disease, portal hypertension, congenital heart diseases, HIV infection, alveolar hypoventilation and others. Accordingly, in particular embodiments, the subject to be treated according to the present invention suffering from pulmonary hypertension or being at risk of developing pulmonary hypertension is diagnosed with a (pre- or coexisting) lung and heart disease or other risk factor as described above.

In some embodiments, said pulmonary hypertension is a pulmonary arterial hypertension, in particular pulmonary arterial hypertension as defined in WHO Group I pulmonary hypertension. In other embodiments, the pulmonary hypertension is pulmonary hypertension associated with interstitial lung disease (PH-ILD) as defined in WHO Group III.

In addition to the method according to the first aspect of the invention as defined above, in a second aspect the invention relates to a pharmaceutical product comprising Treprostinil or a pharmaceutically acceptable salt or derivative thereof for use in the treatment of pulmonary hypertension in a subject being diagnosed with pulmonary hypertension or for use in the prevention of pulmonary hypertension in a subject being at risk of developing pulmonary hypertension, wherein the treatment or prevention comprises the steps of
i) administering to said subject a first liquid composition comprising Treprostinil or a pharmaceutically acceptable salt or derivative thereof in a first concentration in aerosolized form for a first treatment period; and
ii) administering to said subject a second liquid composition comprising Treprostinil or a pharmaceutically acceptable salt or derivative thereof in a second concentration in aerosolized form for a second treatment period following the first treatment period;

wherein the second concentration of Treprostinil or a pharmaceutically acceptable salt or derivative thereof in the second liquid composition is higher than the first concentration of Treprostinil or a pharmaceutically acceptable salt or derivative thereof in the first liquid composition; and
wherein the first and the second liquid compositions are aerosolized and administered to the subject using a soft mist inhaler.

In a third aspect, the invention further relates to Treprostinil or a pharmaceutically acceptable salt or derivative thereof for use in a method of treatment of pulmonary hypertension in a subject being diagnosed with pulmonary hypertension or for use in a method of prevention of pulmonary hypertension in a subject being at risk of developing pulmonary hypertension, wherein the method comprises the steps of
i) administering to said subject a first liquid composition comprising Treprostinil or a pharmaceutically acceptable salt or derivative thereof in a first concentration in aerosolized form for a first treatment period, and
ii) administering to said subject a second liquid composition comprising Treprostinil or a pharmaceutically acceptable salt or derivative thereof in a second concentration in aerosolized form for a second treatment period following the first treatment period;

wherein the second concentration of Treprostinil or a pharmaceutically acceptable salt or derivative thereof in the second liquid composition is higher than the first concentration of Treprostinil or a pharmaceutically acceptable salt or derivative thereof in the first liquid composition; and
wherein the first and the second liquid compositions are aerosolized and administered to the subject using a soft mist inhaler.

In a fourth aspect, the present invention provides for a method of treatment of pulmonary hypertension in a subject being diagnosed with pulmonary hypertension or for a method for the prevention of pulmonary hypertension in a subject being at risk of developing pulmonary hypertension, wherein the method comprises the steps of
i) administering to said subject a first liquid composition comprising Treprostinil or a pharmaceutically acceptable salt or derivative thereof in a first concentration in aerosolized form for a first treatment period, and
ii) administering to said subject a second liquid composition comprising Treprostinil or a pharmaceutically acceptable salt or derivative thereof in a second concentration in aerosolized form for a second treatment period following the first treatment period;

wherein the second concentration of Treprostinil or a pharmaceutically acceptable salt or derivative thereof in the second liquid composition is higher than the first concentration of Treprostinil or a pharmaceutically acceptable salt or derivative thereof in the first liquid composition; and
wherein the first and the second liquid compositions are aerosolized and administered to the subject using a soft mist inhaler.

The embodiments of the invention discussed below apply each to the methods of the invention, the pharmaceutical product or Treprostinil for use in a method of treatment of pulmonary hypertension as defined above as well as to the kit as defined further below.

In particular embodiments, the concentration of Treprostinil or the pharmaceutically acceptable salt or derivative thereof in the first liquid composition is sufficient that the dosage required during the first treatment period can be administered with a small number of inhalation breaths necessary for the inhalation of the complete dose (to be administered in the first treatment period), such as by about 1 to about 3 breaths or by about 2 or 3 breaths or in a single breath by inhalation.

In further particular embodiments, the concentration of Treprostinil or the pharmaceutically acceptable salt or derivative thereof in the second liquid composition is sufficient that the dosage required during the second treatment period can be administered with a small number of inhalation breaths necessary for the inhalation of the complete dose, such as by about 1 to about 3 breaths or by about 2 or 3 breaths or in a single breath by inhalation. In yet further particular embodiments, the concentration of Treprostinil or the pharmaceutically acceptable salt or derivative thereof in an optional third liquid composition is sufficient that the dosage required during the optional third treatment period can be administered with a small number of inhalation breaths necessary for the inhalation of the complete dose, such as by about 1 to about 3 breaths or by about 2 or 3 breaths or in a single breath by inhalation.

Preferably, the concentration of Treprostinil or a pharmaceutically acceptable salt or derivative thereof in the second liquid composition is from about 1.5 to about 2.5 or from about 1.75 to about 2.25 times higher than the concentration of the first liquid composition or, in other words, the concentration of Treprostinil or a pharmaceutically acceptable salt or derivative thereof in the second liquid composition is from about 50% to about 150% or from about 75% to about 125% higher than the concentration of the first liquid composition. In particular embodiments, the concentration of the second liquid composition is about double the concentration of the first liquid composition.

The common therapy for pulmonal hypertension involves slowly increasing the dosage (the so called "titration" effect) of Treprostinil or a pharmaceutically acceptable salt or derivative thereof until Treprostinil or a pharmaceutically acceptable salt or derivative thereof is administered in a final maintenance dose, which is usually a third dosage, but may also be the second dosage.

Accordingly, in preferred embodiment, the invention relates to a method or pharmaceutical product for use in a method as defined above or Treprostinil or a pharmaceutically acceptable salt or derivative thereof for use in a method of treatment of pulmonary hypertension as defined above, further comprising the step of
iii) administering a third liquid composition comprising Treprostinil or a pharmaceutically acceptable salt or derivative thereof in a third concentration in aerosolized form for a third treatment period following the second treatment period;
wherein the third concentration of Treprostinil or a pharmaceutically acceptable salt or derivative thereof in the third liquid composition is higher than the second concentration of Treprostinil or a pharmaceutically acceptable salt or derivative thereof in the second liquid composition, and
wherein the third liquid composition is aerosolized and administered to the subject using a soft mist inhaler.

In particular embodiments, the concentration of Treprostinil or a pharmaceutically acceptable salt or derivative thereof in the third liquid composition is from about 2 to about 4 or from about 2.5 to about 3.5 times higher than the concentration of the first liquid composition or, in other words, the concentration of Treprostinil or a pharmaceutically acceptable salt or derivative thereof in the optional third liquid composition is from about 100% to about 300% or from about 150% to about 250% higher than the concentration of the first liquid composition.

In yet further embodiments, the concentration of Treprostinil or a pharmaceutically acceptable salt or derivative thereof in the second liquid composition amounts to from about 150% to about 250%, or from about 175% to about 225%, or from about 190% to about 210% compared to the amount present in the first liquid composition.

In yet further embodiments, the concentration of Treprostinil or a pharmaceutically acceptable salt or derivative thereof in the optional third liquid composition amounts to from about 200% to about 400%, or from about 250% to about 350%, or from about 290% to about 310% compared to the amount present in the first liquid composition.

In some embodiments, the third concentration of Treprostinil or a pharmaceutically acceptable salt or derivative thereof in the third liquid composition is about three times the first concentration of Treprostinil or a pharmaceutically acceptable salt or derivative thereof.

An advantage of the present invention is the significantly reduced number of required breaths during inhalation compared to the present treatment methods, which require up to nine breaths or even up to 12 breaths once the maintenance dosage of the treatment is reached. Further, in some embodiments, the method according to invention allows to maintain the same posology of one treatment session or, in other words, of the administration of the entire dose to be administered with one actuation of the soft mist inhaler per event during the entire treatment. By using liquid compositions having a first concentration, a second concentration and an optional third concentration of Treprostinil or a pharmaceutically acceptable salt or derivative thereof, it is possible to reduce the number of breaths during the inhalation process significantly. Preferably, the method and other aspects of the invention comprises that the liquid composition is administered with the same number of breaths for the first, second and optional third liquid composition. In a particular embodiment of the invention, the method comprises that the first, the second and optional third liquid compositions are aerosolized and administered using a soft mist inhaler and are administered with one breath (each) per dose.

In further embodiments, however, it is also possible that the dose of Treprostinil or a pharmaceutically acceptable salt or derivative thereof administered to the subject is increased during a treatment period. In these cases, the (increased) dose of Treprostinil or a pharmaceutically acceptable salt or derivative thereof may be administered by an increased number of actuations of the soft mist inhaler per event.

In further particular embodiments, the method and other aspects of the invention comprise that the first, the second and optional third liquid compositions are aerosolized and administered using the same soft mist inhaler, whereas the term "the same" as used in this context refers to the very same soft mist inhaler or to another specimen of the same soft mist inhaler. This has been proven especially beneficial and surprising as the generation of an aerosol of the first, second and optional third liquid composition comprising significantly different (increasing) amounts of dissolved Treprostinil or a pharmaceutically acceptable salt or derivative thereof which was not to be expected in view of the different physico-chemical properties of these liquid compositions.

The (absolute) concentrations of Treprostinil or a pharmaceutically acceptable salt or derivative thereof in the first, second and optional third concentration should be sufficient to provide a sufficient amount of Treprostinil or a pharmaceutically acceptable salt or derivative thereof with the administration in a few breaths, such as in about one to about 5 or to about 4 or to about 3 breaths, preferably in one breath, for the administration of one dose when administered using a soft mist inhaler.

As such, in particular embodiments, the invention relates to a method or pharmaceutical product for use in a method as defined above or Treprostinil or a pharmaceutically acceptable salt or derivative thereof for use in a method of treatment or prevention of pulmonary hypertension as defined above, wherein the concentration of Treprostinil or a pharmaceutically acceptable salt or derivative thereof in the first liquid composition is selected within a range of from about 1 mg/mL to about 5 mg/mL or to about 3 mg/mL. In some embodiments, the first concentration is selected within a range from about 0.8 mg/mL to about 2 mg/mL or from about 1 mg/mL to about 2 mg/mL. In particular embodiments, the first concentration is selected within a range of from about 1.2 mg/mL to about 1.9 mg/mL or from about 1.4 mg/mL to about 1.8 mg/mL, preferably from about 1.5 mg/mL to about 1.7 mg/mL, for example about 1.6 mg/mL.

In further particular embodiments, the invention relates to a method or pharmaceutical product for use in a method as defined above or Treprostinil or a pharmaceutically acceptable salt or derivative thereof for use in a method of treatment or prevention of pulmonary hypertension as defined above, wherein the concentration of Treprostinil or a pharmaceutically acceptable salt or derivative thereof in the second liquid composition is selected within a range of from about 1.6 mg/L to about 6 mg/mL or of from about 2 mg/mL to about 6 mg/mL. Preferably, as outlined above, the concentration of Treprostinil in the second liquid composition is about twice the concentration of the first liquid composition. In some embodiments, the concentration of the second liquid composition is selected within a range from about 1.6 mg/mL or from about 1.8 mg/mL or of from about 2 mg/mL to about 3.8 mg/mL or to about 4 mg/mL. In particular embodiments, the concentration is within a range from about 2.8 mg/mL to about 3.6 mg/mL, preferably from about 3 mg/mL to about 3.4 mg/mL, for example about 3.2 mg/mL.

In further particular embodiments, the invention relates to a method or pharmaceutical product for use in a method as defined above or Treprostinil or a pharmaceutically acceptable salt or derivative thereof for use in a method of treatment or prevention of pulmonary hypertension as defined above, wherein the concentration of Treprostinil or a pharmaceutically acceptable salt or derivative thereof in the optional third liquid composition is selected within a range of from about 2.4 mg/mL to about 9 mg/mL or from about 3 mg/mL to about 9 mg/mL. Preferably, as outlined above, the concentration of Treprostinil in the optional third liquid composition is about three times the concentration of the first liquid composition. In some embodiments, the concentration of the third liquid composition is selected within a range from about 2.4 mg/mL to about 6 mg/mL or from about 2.6 mg/mL or from about 2.8 mg/mL or from about 3 mg/mL to about 6 mg/mL. In particular embodiments, the concentration of Treprostinil in the optional third liquid composition is selected within a range from about 3.2 mg/mL to about 5.0 mg/mL, preferably from about 4.2 mg/mL to about 5.0 mg/mL, for example 4.9 mg/mL.

In a particular embodiment, the invention relates to a method or pharmaceutical product for use in a method as defined above or Treprostinil or a pharmaceutically acceptable salt or derivative thereof for use in a method of treatment or prevention of pulmonary hypertension as defined above, wherein the concentration of Treprostinil or a pharmaceutically acceptable salt or derivative thereof in the first liquid composition is selected within a range of from about 1 mg/mL to about 3 mg/mL, and the concentration of Treprostinil in the second liquid composition is about twice the concentration of Treprostinil in the first liquid composition. In some embodiments, the method or pharmaceutical product comprises an optional third liquid composition as defined above, wherein the concentration of Treprostinil in the third liquid composition is about three times the concentration of Treprostinil in the first liquid composition.

In a particular embodiment, the invention relates to a method or pharmaceutical product for use in a method as defined above or Treprostinil or a pharmaceutically acceptable salt or derivative thereof for use in a method of treatment or prevention of pulmonary hypertension as defined above, wherein the method comprises:
i) administering to a subject a first liquid composition comprising Treprostinil or a pharmaceutically acceptable salt or derivative thereof in a first concentration in aerosolized form for a first treatment period;
ii) administering to said subject a second liquid composition comprising Treprostinil or a pharmaceutically acceptable salt or derivative thereof in a second concentration in aerosolized form for a second treatment period following the first treatment period; and optionally
iii) administering a third liquid composition comprising Treprostinil or a pharmaceutically acceptable salt or derivative thereof in a third concentration in aerosolized form for a third treatment period following the second treatment period;
wherein the concentration of Treprostinil or a pharmaceutically acceptable salt or derivative thereof in the first liquid composition is selected within a range of from about 1.2 mg/mL to about 1.9 mg/mL or from about 1.5 mg/mL to about 1.7 mg/mL; and wherein the concentration of Treprostinil or a pharmaceutically acceptable salt or derivative thereof in the second liquid composition is selected within the range of from about 2.4 mg/mL to about 3,8 mg/mL or from about 3 mg/mL to about 3.4 mg/mL, or is about twice the concentration of Treprostinil or a pharmaceutically acceptable salt or derivative thereof in the first composition; and wherein the concentration of Treprostinil or a pharmaceutically acceptable salt or derivative thereof in the optional third liquid composition is selected within the range of from about 3.6 mg/mL to about 5.7 mg/mL or from about 4.2 mg/mL to about 4.9 mg/mL, or is about three times the concentration of Treprostinil or a pharmaceutically acceptable salt or derivative thereof in the first composition.

In some embodiments, the invention relates a method or pharmaceutical product for use in a method as defined above or Treprostinil or a pharmaceutically acceptable salt or derivative thereof for use in a method of treatment or prevention of pulmonary hypertension as defined above, wherein the amount of Treprostinil or a pharmaceutically acceptable salt or derivative thereof administered in a single event (i.e. upon a single activation of the soft mist inhaler) during the first treatment period is selected with the range of from about 10 µg to about 25 µg, preferably of from about 15 µg to about 20 µg. In these embodiments, the first liquid composition preferably is aerosolized in an amount of from about 8.8 µL to about 13.3 µL when provided in a concentration of from about 1.5 to about 1.7 mg/mL. In a particular embodiment, the amount of Treprostinil or a pharmaceutically acceptable salt or derivative thereof administered in a single event during the first treatment period is about 18 µg.

In some embodiments, the invention relates to a method or pharmaceutical product for use in a method as defined above or Treprostinil or a pharmaceutically acceptable salt or derivative thereof for use in a method of treatment or prevention of pulmonary hypertension as defined above, wherein the amount of Treprostinil or a pharmaceutically acceptable salt or derivative thereof administered in a single event during the second treatment period is selected with the range of from about 25 µg to about 50 µg, preferably of from about 30 µg to about 40 µg. In these embodiments, the second liquid composition preferably is aerosolized and administered in an amount of from about 8.8 µL to about 13.3 µL when provided in a concentration of from about 3.0 to about 3.4 mg/mL. In a particular embodiment, the amount of Treprostinil or a pharmaceutically acceptable salt or derivative thereof administered in a single event during the second treatment period is about 36 µg.

In some embodiments, the invention relates a method or pharmaceutical product for use in a method as defined above or Treprostinil or a pharmaceutically acceptable salt or derivative thereof for use in a method of treatment or prevention of pulmonary hypertension as defined above, wherein the amount of Treprostinil or a pharmaceutically acceptable salt or derivative thereof administered in a single event during the optional third treatment period is selected with the range of from about 40 µg to about 70 µg, preferably of from about 45 µg to about 60 µg. In these embodiments, the optional third liquid composition preferably is aerosolized and administered in an amount of from about 8.8 µL to about 13.3 µL when provided in a concentration of from about 4.5 to about 4.9 mg/mL. In a particular embodiment, the amount of Treprostinil or a pharmaceutically acceptable salt or derivative thereof administered in a single event during the optional third treatment period is about 54 µg.

In some embodiments, the invention relates to a method or pharmaceutical product for use in a method as defined above or Treprostinil or a pharmaceutically acceptable salt or derivative thereof for use in a method of treatment or prevention of pulmonary hypertension as defined above, wherein the amount of Treprostinil or a pharmaceutically acceptable salt or derivative thereof to be administered in a single event during the first, second and optional third treatment period as defined above is administered in one breath each upon a single activation of the soft mist inhaler upon which a single metered dose of Treprostinil or a pharmaceutically acceptable salt or derivative is dispensed.

In general, in the treatment or prevention of pulmonary hypertension Treprostinil or a pharmaceutically acceptable salt or derivative thereof is often administered four times a day (i.e. in four events) with usually about 4 hours between each administration. As such, the total daily dosage of Treprostinil or a pharmaceutically acceptable salt or derivative thereof is about four times the dosage of a single dose as administered in a single event.

Accordingly, in some embodiments, the invention relates to a method or pharmaceutical product for use in a method as defined above or Treprostinil or a pharmaceutically acceptable salt or derivative thereof for use in a method of treatment or prevention of pulmonary hypertension as defined above, wherein the Treprostinil or a pharmaceutically acceptable salt or derivative thereof is administered to the subject during the first treatment period in a daily dose selected within a range of from about 40 µg to about 100 µg, such as from about 60 µg to about 80 µg. In a particular embodiment, the Treprostinil or a pharmaceutically acceptable salt or derivative thereof is administered to the subject during the first treatment period in a daily dose of about 72 µg.

In further embodiments, the invention relates to a method or pharmaceutical product for use in a method as defined above or Treprostinil or a pharmaceutically acceptable salt or derivative thereof for use in a method of treatment or prevention of pulmonary hypertension as defined above, wherein the Treprostinil or a pharmaceutically acceptable salt or derivative thereof is administered to the subject during the second treatment period in a daily dose selected within a range of from about 100 µg to about 200 µg or from about 130 µg to about 170 µg. In a particular embodiment, the Treprostinil or a pharmaceutically acceptable salt or derivative thereof is administered to the subject during the second treatment period in a daily dose of about 144 µg.

In yet further embodiments, the invention relates to a method or pharmaceutical product for use in a method as defined above or Treprostinil or a pharmaceutically acceptable salt or derivative thereof for use in a method of treatment or prevention of pulmonary hypertension as defined above, wherein the Treprostinil or a pharmaceutically acceptable salt or derivative thereof is administered to the subject during the optional third treatment period in a daily dose selected within a range of from about 160 µg to about 280 µg or from about 200 mg to about 240 µg. In a particular embodiment the Treprostinil or a pharmaceutically acceptable salt or derivative thereof is administered to the subject during the third treatment period in a daily dose of about 216 µg.

The first and second treatment period as defined above usually last for about two weeks, before the final third treatment period is reached. The third treatment period is considered a maintenance period and is usually lasting indefinitely.

As such, in some embodiments, the invention relates a method or pharmaceutical product for use in a method as defined above or Treprostinil or a pharmaceutically acceptable salt or derivative thereof for use in a method of treatment or prevention of pulmonary hypertension as defined above, wherein the duration of the first treatment period is selected within the range of from about 1 day to about 15 days. In some embodiments, the duration of the first treatment period is at least about 10 days, preferably 10 or 12 to 15 days, in particular 14 days.

The duration of the second treatment period is, in some embodiments, about as long as the first treatment period. Accordingly, in some embodiments, the invention relates to a method or pharmaceutical product as defined above, wherein the duration of the second treatment period is selected within the range of from about 1 day to about 15 days. In some embodiments, the duration of the second treatment period is at least about 10 days, preferably 10 or 12 to 15 days, in particular 14 days.

In some embodiments, depending on the specific needs of the particular subject treated, the second treatment period may be extended and then turns into a maintenance period. In these cases, there is no third treatment period. Preferably, however, the method or pharmaceutical product for use in a method as defined above comprises a third treatment period, which is a maintenance period. Said maintenance period may last indefinitely or as long as such treatment is needed by the subject.

The liquid compositions comprising Treprostinil or a pharmaceutically acceptable salt or derivative thereof may be any suitable liquid composition that is suitable and pharmaceutically acceptable for inhalation using a soft mist inhaler. The compositions may be 100 % aqueous or alcohol based, in particular ethanol based. The compositions used in the invention may comprise any suitable excipients, adjuvants, stabilizers and preservatives or may be free of them.

Accordingly, in one embodiment, the invention relates to a method or pharmaceutical product for use in a method as defined above or Treprostinil or a pharmaceutically acceptable salt or derivative thereof for use in a method of treatment or prevention of pulmonary hypertension as defined above, wherein the first, the second and the optional third liquid composition each comprise a liquid vehicle in which the Treprostinil or a pharmaceutically acceptable salt or derivative thereof is dissolved.

In particular embodiments, the liquid vehicle may comprise one or more inorganic or organic solvents, preferably solvents which are physiologically acceptable and which are considered acceptable for pulmonary application by inhalation. In some embodiments, the liquid vehicle of the first, second or third liquid composition may each be an aqueous solution comprising an aqueous solvent or a mixture of solvents. Suitable solvents include water, ethanol, propylene glycol as well as binary or tertiary mixtures thereof. In some embodiments, the liquid compositions may comprise mixtures of water and an alcohol such as ethanol or propylene glycol, preferably ethanol, as the liquid vehicle, for example mixtures comprising from about 99 % (v/v) to about 1 % (v/v) or from about 95 % (v/v) to about 10 % (v/v) or from about 90 % (v/v) to about 50 % (v/v) water and the corresponding amount (adding to 100 % (v/v)) of an alcohol, preferably ethanol. In particular embodiments, the liquid compositions comprise water as the liquid vehicle (comprising no further inorganic or organic solvents).

In further particular embodiments, the first and the second liquid composition as well as the optional third liquid composition may all have the same liquid vehicle. In further embodiments, however, two of the three liquid compositions, for example the first and the second liquid compositions, may have the same or substantially the same liquid vehicle while one of the three liquid compositions, for example the third liquid composition, may have a different liquid vehicle, for example, comprising different solvents or the same solvents in a different ratio. In yet further particular embodiments, both or all three liquid compositions, as the case may be, may have a different liquid vehicle, for example, comprising different solvents or the same solvents in a different ratio.

In some embodiments, the first, the second and the optional third liquid composition of the method or pharmaceutical product for use in a method as defined above further comprises one or more pharmaceutically acceptable excipients. Given the pH-dependency of the solubility of Treprostinil or its salts in aqueous solvent systems the first, the second and the optional third liquid composition may comprise pharmaceutically acceptable buffer agents such as sodium citrate or others known to those of skill in the art. For the long-term stability of the pharmaceutical product, pharmaceutically acceptable chelating agents such as EDTA or others may also be comprised. Furthermore, the first, the second and the optional third liquid composition may comprise a pharmaceutically acceptable preservative, for example a cationic surfactant such as benzalkonium chloride (as the product is intended to be multidose sterile).

Treprostinil may be used as the free acid or in form of a pharmaceutically acceptable salt or derivative thereof. Suitable salts or derivatives of Treprostinil are known to the skilled person and comprise, but are not limited to Treprostinil sodium and Treprostinil diethanolamine as described above. Suitable derivatives of Treprostinil comprise, but are not limited to carboxylic esters of Treprostinil such as Treprostinil Palmitil as described above. It is preferred that Treprostinil is used in the same form, during every treatment period.

Accordingly, in particular embodiments, the invention relates to a method or pharmaceutical product for use in a method as defined above or Treprostinil or a pharmaceutically acceptable salt or derivative thereof for use in a method of treatment or prevention of pulmonary hypertension as defined above, or to the kits as defined below wherein the Treprostinil or a pharmaceutically acceptable salt or derivative thereof in the first, the second and the optional third liquid composition is Treprostinil sodium or Treprostinil diethanolamine (diolamine), specifically Treprostinil sodium.

The present invention utilizes a soft mist inhaler or, in other words, a soft mist inhalation device for the administration of Treprostinil or a pharmaceutically acceptable salt or derivative thereof. Suitable soft most inhalers are known to the skilled person and are described, for example in US 2014/076308 A1 or WO 2018/197730 A1. Several different types of soft mist inhalers are available, which are based on different nebulization methods. Some soft mist inhalers are based on vibrating membranes, for example Philips InnoSpire and others, while other soft mist inhalers utilize pressurized liquids and/or propellants to aerosolize the compositions.

In the context of the present invention the liquid compositions of the first, second and optional third liquid composition comprising Treprostinil or a pharmaceutically acceptable salt or derivative thereof, in particular embodiments, have a mass medium aerodynamic diameter (MMAD) of from about 1 µm to about 10 µm or from about 2 µm to about 8 µm or from about 4 µm to about 6 µm when aerosolized using a soft mist inhaler.

In particular, the invention relates to a method or pharmaceutical product for use in a method as defined above or Treprostinil or a pharmaceutically acceptable salt or derivative thereof for use in a method of treatment or prevention of pulmonary hypertension as defined above, wherein the soft mist inhaler is adapted to generate an aerosol or plume of aerosol of the first, second and optional third liquid composition having an average velocity of 1.0 to 2.5 m/s, preferably of 1.3 to 2.1 m/s.

In preferred embodiments, the invention relates to a method or pharmaceutical product for use in a method as defined above or Treprostinil or a pharmaceutically acceptable salt or derivative thereof for use in a method of treatment prevention of pulmonary hypertension as defined above, wherein the soft mist inhaler is adapted to hold the first, second and the optional third liquid composition comprising Treprostinil or a pharmaceutically acceptable salt or derivative thereof at atmospheric pressure (in non-pressurized form). Preferably, soft mist the inhaler also does not utilize a propellant gas (such as a hydrofluoroalkane (HFA) e.g. HFA-134a or HFA-227) to aerosolize or nebulize the respective liquid composition.

Preferred inhalers to be used in the context of the present invention are pure mechanical and manual soft mist inhalers, which do not utilize electrical means to nebulize the compositions.

Accordingly, in some preferred embodiments, the invention relates to a method or pharmaceutical product for use in a method as defined above or Treprostinil or a pharmaceutically acceptable salt or derivative thereof for use in a method of treatment or prevention of pulmonary hypertension as defined above, wherein the soft mist inhaler is adapted to aerosolize the first, second and optional third liquid composition comprising Treprostinil or a pharmaceutically acceptable salt or derivative thereof by an entirely mechanical mechanism, preferably by a mechanism that does not comprise electronic means.

Mechanical soft mist inhalers that do not depend on pressurized aerosols or electrical means to nebulize a composition often use a specific nozzle to nebulize the liquid composition. Such nozzles include but are not limited to nozzles that are based on Raleigh principles, or swirl nozzles or impingement type nozzles.

In preferred embodiments, the invention relates to a method or pharmaceutical product for use in a method as defined above or Treprostinil or a pharmaceutically acceptable salt or derivative thereof for use in a method of treatment or prevention of pulmonary hypertension as defined above, wherein the soft mist inhaler comprises a nozzle for the aerosolization of the first, second and optional third liquid composition comprising Treprostinil or a pharmaceutically acceptable salt or derivative thereof.

In particular preferred embodiments, the soft mist inhaler comprises a pumping unit adapted to generate a working pressure of the first, second and optional third liquid composition comprising Treprostinil or a pharmaceutically acceptable salt or derivative thereof within the range of from about 50 to about 350 bar, or in the range of from about 50 bar to about 250 bar or from about 100 to about 250 bar. Preferably, the pumping unit is mechanical and operated manually by the user.

In particular embodiments of the invention, the soft mist inhaler is an impingement-type soft mist inhaler having at least one nozzle with at least two ejection channels adapted to generate at least two jets of the first, second and optional third liquid composition comprising Treprostinil or a pharmaceutically acceptable salt or derivative thereof that intersect to generate an aerosol. In further particular embodiments, the at least two ejection channels are adapted or oriented such that the at least two liquid jets ejected therefrom impinge to generate an aerosol of the respective liquid composition. In further particular embodiments, the impinge-type soft mist inhaler has two ejection channels. In yet further embodiments, the ejection channels have a diameter in the range of from about 4 µm to about 15 µm or from about 6 µm to about 12 µm or from about 8 µm to about 10 µm.

In preferred embodiments of the invention, the soft mist inhaler is a hand-held inhalation device, i.e. a device which can be held and operated with a single hand or with both hands of a user, for delivering a nebulised medically active aerosol for inhalation therapy, comprising
(a) a housing having a user-facing side;
(b) an impingement-type nozzle for generating the nebulised aerosol by collision of at least two liquid jets, the nozzle being firmly affixed to the user-facing side of the housing such as to be immobile relative to the housing;
(c) a fluid reservoir arranged within the housing; and
(d) a pumping unit arranged within the housing, the pumping unit having
   - an upstream end that is fluidically connected to the fluid reservoir;
   - a downstream end that is fluidically connected to the nozzle;
wherein the pumping unit is adapted for pumping fluid from the fluid reservoir to the nozzle;
wherein the pumping unit further comprises
(i) a riser pipe having an upstream end, wherein the riser pipe is
   - adapted to function as a piston in the pumping unit, and
   - firmly affixed to the user-facing side of the housing such as to be immobile relative to the housing; and
(ii) a hollow cylinder located upstream of the riser pipe, wherein the upstream end of the riser pipe is inserted in the cylinder such that the cylinder is longitudinally movable on the riser pipe;
(iii) a lockable means for storing potential energy when locked and for releasing the stored energy when unlocked, the means being arranged outside of, and mechanically coupled to, the cylinder such that unlocking the means results in a propulsive longitudinal movement of the cylinder towards the downstream end of the pumping unit.

Such an inhalation device is, for example described in WO 2018/197730 A1, the contents of which are incorporated herein in their entirety by reference. An advantage of such soft mist inhalation devices operating with a purely mechanical source of pressure is, for example, that such soft mist inhalation devices allow for the aerosolization of a broad variety of liquid compositions with different liquid vehicles and concentrations of the dissolved actives such as Treprostinil or a pharmaceutically acceptable salt or derivative thereof resulting in a broad variability of physical properties which are relevant for aerosolization, especially when aerosolized with an impingement-type nozzle, such as the density, the viscosity, the vapor pressure, and/or the surface tension.

In a fifth aspect, the invention further relates to a kit comprising a soft mist inhaler and said first, second and optional third liquid composition comprising Treprostinil or a pharmaceutically acceptable salt or derivative thereof. Said kit preferably further comprises instructions for performing said method, in particular instructions on the administration of the liquid compositions comprising Treprostinil or a pharmaceutically acceptable salt or derivative thereof. It should be noted that all embodiments or features as disclosed in connection with the method or pharmaceutical product for use in a method as defined above or Treprostinil or a pharmaceutically acceptable salt or derivative thereof for use in a method of treatment or prevention of pulmonary hypertension according to the first to fourth aspects of the invention as defined above equally apply to the kit of this fifth aspect of the invention.

Accordingly, the invention further relates to a kit comprising
a) a first liquid composition comprising Treprostinil or a pharmaceutically acceptable salt or derivative thereof in a first concentration; and
b) a second liquid composition comprising Treprostinil or a pharmaceutically acceptable salt or derivative thereof in a second concentration; and optionally
c) a third liquid composition comprising Treprostinil or a pharmaceutically acceptable salt or derivative thereof in a third concentration;
wherein the second concentration of Treprostinil or a pharmaceutically acceptable salt or derivative thereof in the second liquid composition is higher than the first concentration of Treprostinil or a pharmaceutically acceptable salt or derivative thereof in the first liquid composition and wherein the third concentration of Treprostinil or a pharmaceutically acceptable salt or derivative thereof in the optional third liquid composition is higher than the second concentration of Treprostinil or a pharmaceutically acceptable salt or derivative thereof in the second liquid composition.

The first, second and third liquid compositions comprising Treprostinil or a pharmaceutically acceptable salt or derivative thereof are as defined above for the method or pharmaceutical product according to the first to fourth aspect of the invention.

As such, the first concentration of Treprostinil or a pharmaceutically acceptable salt or derivative thereof in the first liquid composition of the present kit, in some embodiments, is selected within a range of from about of from about 1 mg/mL to about 5 mg/mL or to about 3 mg/mL. In some embodiments, the first concentration is selected within a range from about 0.8 mg/mL to about 2 mg/mL or from about 1 mg/mL to about 2 mg/mL. In particular embodiments, the first concentration is selected within a range of from about 1.2 mg/mL to about 1.9 mg/mL or from about 1.4 mg/mL to about 1.8 mg/mL, preferably from about 1.5 mg/mL to about 1.7 mg/mL, for example about 1.6 mg/mL. In some embodiments, the first concentration is within a range from about 1 mg/mL to about 2 mg/mL. In particular embodiments, the first concentration is within a range from about 1.4 mg/mL to 1.8 mg/mL, preferably 1.5 mg/mL to 1.7 mg/mL.

The second concentration of Treprostinil or a pharmaceutically acceptable salt or derivative thereof in the second liquid composition, in some embodiments, is selected within a range of from about 1.6 mg/L to about 6 mg/mL or of from about 2 mg/mL to about 6 mg/mL. Preferably, as outlined above, the concentration of Treprostinil in the second liquid composition is about twice the concentration of the first liquid composition. In some embodiments, the concentration of the second liquid composition is selected within a range from about 1.6 mg/mL or from about 1.8 mg/mL or of from about 2 mg/mL to about 3.8 mg/mL or to about 4 mg/mL. In particular embodiments, the concentration is within a range from about 2.8 mg/mL to about 3.6 mg/mL, preferably from about 3 mg/mL to about 3.4 mg/mL, for example about 3.2 mg/mL.

The third concentration of Treprostinil or a pharmaceutically acceptable salt or derivative thereof in the optional third liquid composition is selected within a range of from about 2.4 mg/mL to about 9 mg/mL or from about 3 mg/mL to about 9 mg/mL. Preferably, as outlined above, the concentration of Treprostinil in the optional third liquid composition is about three times the concentration of the first liquid composition. In some embodiments, the concentration of the third liquid composition is selected within a range from about 2.4 mg/mL to about 6 mg/mL or from about 2.6 mg/mL or from about 2.8 mg/mL or from about 3 mg/mL to about 6 mg/mL. In particular embodiments, the concentration of Treprostinil in the optional third liquid composition is selected within a range from about 3.2 mg/mL to about 5.0 mg/mL, preferably from about 4.2 mg/mL to about 5.0 mg/mL, for example 4.9 mg/mL.

In a particular embodiment, the invention relates to kit as defined above, wherein the concentration of Treprostinil or a pharmaceutically acceptable salt or derivative thereof in the first liquid composition is selected within a range of from about 1 mg/mL to about 3 mg/mL, in particular about 1.5 mg/mL to 1.7 mg/mL, as defined above, and the concentration of Treprostinil in the second liquid composition is about twice the concentration of Treprostinil in the first liquid composition. In some embodiments, the kit comprises an optional third liquid composition as defined above, wherein the concentration of Treprostinil in the third liquid composition is about three times the concentration of Treprostinil in the first liquid composition.

In order to improve treatment efficiency and ease of use, the liquid compositions comprising Treprostinil or a pharmaceutically acceptable salt or derivative thereof are preferably provided in separate containers or alternatively in separate soft mist inhalers.

Accordingly, in some embodiments, the first, the second and the optional third liquid composition comprising Treprostinil or a pharmaceutically acceptable salt or derivative thereof are provided separately in a first, a second and an optional third container.

In further embodiments, the kit comprises a first, a second and optionally a third soft mist inhaler, wherein each soft mist inhaler comprises one of the first, second and optionally third liquid composition. The compositions are preferably provided in form of cartridges for the soft mist inhaler and may be provided separate or pre-inserted.

Accordingly, in some embodiments, the kit according to the invention comprises a single soft mist inhaler adapted to consecutively receive the first, second and optional third liquid composition and to aerosolize said compositions. In this case, the first, second and optional third liquid composition comprising Treprostinil or a pharmaceutically acceptable salt or derivate thereof are provided in separate containers, wherein the containers preferably are cartridges for the soft mist inhaler.

In a particular embodiment the kit comprises,
a) a first liquid composition comprising Treprostinil or a pharmaceutically acceptable salt or derivative thereof in a first concentration; and
b) a second liquid composition comprising Treprostinil or a pharmaceutically acceptable salt or derivative thereof in a second concentration; and optionally
c) a third liquid composition comprising Treprostinil or a pharmaceutically acceptable salt or derivative thereof in a third concentration; and
d ) a soft mist inhaler;
wherein the second concentration of Treprostinil or a pharmaceutically acceptable salt or derivative thereof in the second liquid composition is higher than the first concentration of Treprostinil or a pharmaceutically acceptable salt or derivative thereof in the first liquid composition and wherein the third concentration of Treprostinil or a pharmaceutically acceptable salt or derivative thereof in the optional third liquid composition is higher than the second concentration of Treprostinil or a pharmaceutically acceptable salt or derivative thereof in the second liquid composition; wherein each liquid composition is provided in a separate container.

In particular embodiments, the containers referred to herein holding the first, second and optional third liquid composition, respectively, are dimensioned to comprise the respective liquid composition in an amount which is sufficient to dispense a plurality of doses of the respective liquid therefrom. In particular embodiments, the container have an inner volume for holing the respective liquid composition of from about 2 mL to about 8 mL or from about 3 mL to about 6 mL or from about 4 mL to about 5 mL. Accordingly, in further preferred embodiments, the containers as referred to herein are adapted for holding at least from about 1 to about 25, or from about 3 to about 15 or from about 5 to about 10 daily doses of the corresponding liquid composition.

In further embodiments, the kits according to this aspect of the present invention comprise a soft mist inhaler which is adapted to aerosolize the first, second and optional third liquid composition comprising Treprostinil or a pharmaceutically acceptable salt or derivative thereof by an entirely mechanical mechanism, preferably by a mechanism that does not comprise electronic means as described above.

In preferred embodiments, the soft mist inhaler comprised by the present kits comprises a pumping unit adapted to generate a working pressure of the first, second and optional third liquid composition comprising Treprostinil or a pharmaceutically acceptable salt or derivative thereof within the range of from about 50 to about 350 bar. Preferably, the pumping unit is mechanical and operated manually by the user.

In further preferred embodiments of the present kits, the soft mist inhaler is an impingement-type soft mist inhaler having at least one nozzle with at least two ejection channels adapted to generate at least two jets of the first, second and optional third liquid composition comprising Treprostinil or a pharmaceutically acceptable salt or derivative thereof that intersect to generate an aerosol. In further preferred embodiments, the at least two ejection channels are adapted or oriented such that the at least two liquid jets ejected therefrom impinge to generate an aerosol of the respective liquid composition.

In yet further preferred embodiments of the present kits, the soft mist inhaler is a hand-held inhalation device for delivering a nebulised medically active aerosol for inhalation therapy, comprising
(a) a housing having a user-facing side;
(b) an impingement-type nozzle for generating the nebulised aerosol by collision of at least two liquid jets, the nozzle being firmly affixed to the user-facing side of the housing such as to be immobile relative to the housing;
(c) a fluid reservoir arranged within the housing; and
(d) a pumping unit arranged within the housing, the pumping unit having
   - an upstream end that is fluidically connected to the fluid reservoir;
   - a downstream end that is fluidically connected to the nozzle;
wherein the pumping unit is adapted for pumping fluid from the fluid reservoir to the nozzle;
wherein the pumping unit further comprises
(i) a riser pipe having an upstream end, wherein the riser pipe is
   - adapted to function as a piston in the pumping unit, and
   - firmly affixed to the user-facing side of the housing such as to be immobile relative to the housing; and
(ii) a hollow cylinder located upstream of the riser pipe, wherein the upstream end of the riser pipe is inserted in the cylinder such that the cylinder is longitudinally movable on the riser pipe;
(iii) a lockable means for storing potential energy when locked and for releasing the stored energy when unlocked, the means being arranged outside of, and mechanically coupled to, the cylinder such that unlocking the means results in a propulsive longitudinal movement of the cylinder towards the downstream end of the pumping unit.

The invention further relates to the following numbered items (list of numbered items 1/2).
1. A method for the inhalative administration of Treprostinil or a pharmaceutically acceptable salt or derivative thereof to a subject in need of such administration, the method comprising the steps of
   i) administering to said subject a first liquid composition comprising Treprostinil or a pharmaceutically acceptable salt or derivative thereof in a first concentration in aerosolized form for a first treatment period, and
   ii) administering to said subject a second liquid composition comprising Treprostinil or a pharmaceutically acceptable salt or derivative thereof in a second concentration in aerosolized form for a second treatment period following the first treatment period;

   wherein the second concentration of Treprostinil or a pharmaceutically acceptable salt or derivative thereof in the second liquid composition is higher than the first concentration of Treprostinil or a pharmaceutically acceptable salt or derivative thereof in the first liquid composition, and
   wherein the first and the second liquid compositions are aerosolized and administered using a soft mist inhaler.
2. The method according to item 1, wherein the subject diagnosed with pulmonary hypertension or is threatened by developing pulmonary hypertension.
3. A pharmaceutical product comprising Treprostinil or a pharmaceutically acceptable salt or derivative thereof for use in the treatment of pulmonary hypertension in a subject being diagnosed with pulmonary hypertension wherein the treatment comprises the steps of
   i) administering to said subject a first liquid composition comprising Treprostinil or a pharmaceutically acceptable salt or derivative thereof in a first concentration in aerosolized form for a first treatment period, and
   ii) administering to said subject a second liquid composition comprising Treprostinil or a pharmaceutically acceptable salt or derivative thereof in a second concentration in aerosolized form for a second treatment period following the first treatment period;
   wherein the second concentration of Treprostinil or a pharmaceutically acceptable salt or derivative thereof in the second liquid composition is higher than the first concentration of Treprostinil or a pharmaceutically acceptable salt or derivative thereof in the first liquid composition, and wherein the first and the second liquid compositions are aerosolized and administered using a soft mist inhaler.
4. The method or pharmaceutical product according to item 1, further comprising the step of
   iii) administering a third liquid composition comprising Treprostinil or a pharmaceutically acceptable salt or derivative thereof in a third concentration in aerosolized form for a third treatment period following the second treatment period;
   wherein the third concentration of Treprostinil or a pharmaceutically acceptable salt or derivative thereof in the third liquid composition is higher than the second concentration of Treprostinil or a pharmaceutically acceptable salt or derivative thereof in the second liquid composition, and wherein the third liquid composition is aerosolized and administered using a soft mist inhaler.
5. The method or pharmaceutical product according to any one of the preceding items, wherein the concentration of Treprostinil or a pharmaceutically acceptable salt or derivative thereof in the first liquid composition is selected within a range of from about 1 mg/mL to about 3 mg/mL.
6. The method or pharmaceutical product according to any one of the preceding items, wherein the concentration of Treprostinil or a pharmaceutically acceptable salt or derivative thereof in the second liquid composition is selected within a range of from about 2 mg/mL to about 4 mg/mL.
7. The method or pharmaceutical product according to any one of the preceding items, wherein the concentration of Treprostinil or a pharmaceutically acceptable salt or derivative thereof in the third liquid composition is selected within a range of from about 3 mg/mL to about 6 mg/mL.
8. The method or pharmaceutical product according to any one of the preceding items, wherein the first, the second and the optional third liquid composition each is an aqueous solution comprising an aqueous solvent or mixture of solvents.
9. The method or pharmaceutical product according to any one of the preceding items, wherein the first, the second and the optional third liquid composition further comprises one or more pharmaceutically acceptable excipients.
10. The method or pharmaceutical product according to any one of the preceding items, wherein the duration of the first treatment period is selected within the range of from about 1 day to about 15 days.
11. The method or pharmaceutical product according to any one of the preceding items, wherein the duration of the second treatment period is selected within the range of from about 1 day to about 15 days.
12. The method or pharmaceutical product according to any one of the preceding items, wherein the amount of Treprostinil or a pharmaceutically acceptable salt or derivative thereof administered in a single event during the first treatment period is selected with the range of from about 10 µg to about 25 µg, preferably of from about 15 µg to about 20 µg.
13. The method or pharmaceutical product according to any one of the preceding items, wherein the amount of Treprostinil or a pharmaceutically acceptable salt or derivative thereof administered in a single event during the second treatment period is selected with the range of from about 25 µg to about 50 µg, preferably of from about 30 µg to about 40 µg.
14. The method or pharmaceutical product according to any one of the preceding items, wherein the amount of Treprostinil or a pharmaceutically acceptable salt or derivative thereof administered in a single event during the third treatment period is selected with the range of from about 40 µg to about 70 µg, preferably of from about 45 µg to about 60 µg.
15. The method or pharmaceutical product according to any one of the preceding items, wherein the amount of Treprostinil or a pharmaceutically acceptable salt or derivative thereof to be administered in a single event during the first, second and optional third treatment period is administered in one breath each upon a single activation of the soft mist inhaler upon which a single metered dose of Treprostinil or a pharmaceutically acceptable salt or derivative is dispensed.
16. The method or pharmaceutical product according to any one of the preceding items, wherein the Treprostinil or a pharmaceutically acceptable salt or derivative thereof is administered to the subject during the first treatment period in a daily dose selected within a range of from about 40 µg to about 100 µg.
17. The method or pharmaceutical product according to any one of the preceding items, wherein the Treprostinil or a pharmaceutically acceptable salt or derivative thereof is administered to the subject during the second treatment period in a daily dose selected within a range of from about 100 µg to about 200 µg.
18. The method or pharmaceutical product according to any one of the preceding items, wherein the Treprostinil or a pharmaceutically acceptable salt or derivative thereof is administered to the subject during the third treatment period in a daily dose selected within a range of from about 160 µg to about 280 µg.
19. The method or pharmaceutical product according to any one of the preceding items, wherein the Treprostinil or a pharmaceutically acceptable salt or derivative thereof in the first, the second and the optional third liquid composition is Treprostinil sodium.
20. The method or pharmaceutical product according to any one of the preceding items, wherein the soft mist inhaler is adapted to generate an aerosol or plume of aerosol of the first, second and optional third liquid composition having an average velocity of 1.0 to 2.5m/s, preferably of 1.3 to 2.1 m/s.
21. The method or pharmaceutical product according to any one of the preceding items, wherein the soft mist inhaler is adapted to hold the first, second and the optional third liquid composition comprising Treprostinil or a pharmaceutically acceptable salt or derivative thereof at atmospheric pressure (in non-pressurized form).
22. The method or pharmaceutical product according to any one of the preceding items, wherein the soft mist inhaler is adapted to aerosolize the first second and optional third liquid composition comprising Treprostinil or a pharmaceutically acceptable salt or derivative thereof by an entirely mechanical mechanism, preferably by a mechanism that does not comprise electronic means.
23. The method or pharmaceutical product according to any one of the preceding items, wherein the soft mist inhaler comprises a nozzle for the aerosolization of the first second and optional third liquid composition comprising Treprostinil or a pharmaceutically acceptable salt or derivative thereof.
24. The method or pharmaceutical product according to item 23, wherein the soft mist inhaler comprises a pumping unit adapted to generate a working pressure of the first second and optional third liquid composition comprising Treprostinil or a pharmaceutically acceptable salt or derivative thereof within the range of from about 50 to about 350 bar.
25. The method or pharmaceutical product according to any one of the preceding items, wherein the soft mist inhaler is an impingement-type soft mist inhaler having at least one nozzle with at least two ejection channels adapted to generate at least two jets of the first, second and optional third liquid composition comprising Treprostinil or a pharmaceutically acceptable salt or derivative thereof that intersect to generate an aerosol.
26. The method or pharmaceutical product according to any one of the preceding items, wherein the soft mist inhaler is a hand-held inhalation device for delivering a nebulised medically active aerosol for inhalation therapy, comprising
   (a) a housing having a user-facing side;
   (b) an impingement-type nozzle for generating the nebulised aerosol by collision of at least two liquid jets, the nozzle being firmly affixed to the user-facing side of the housing such as to be immobile relative to the housing;
   (c) a fluid reservoir arranged within the housing; and
   (d) a pumping unit arranged within the housing, the pumping unit having
      - an upstream end that is fluidically connected to the fluid reservoir;
      - a downstream end that is fluidically connected to the nozzle;

   wherein the pumping unit is adapted for pumping fluid from the fluid reservoir to the nozzle;
   wherein the pumping unit further comprises
      (i) a riser pipe having an upstream end, wherein the riser pipe is
         - adapted to function as a piston in the pumping unit, and
         - firmly affixed to the user-facing side of the housing such as to be immobile relative to the housing; and
      (ii) a hollow cylinder located upstream of the riser pipe, wherein the upstream end of the riser pipe is inserted in the cylinder such that the cylinder is longitudinally movable on the riser pipe;
      (iii) a lockable means for storing potential energy when locked and for releasing the stored energy when unlocked, the means being arranged outside of, and mechanically coupled to, the cylinder such that unlocking the means results in a propulsive longitudinal movement of the cylinder towards the downstream end of the pumping unit.
27. A method of treating pulmonary hypertension in a subject suffering from pulmonary hypertension comprising the method for the inhalative administration of Treprostinil or a pharmaceutically acceptable salt or derivative thereof according to item 1.
28. The methods or the pharmaceutical product according any one of the preceding items, wherein the pulmonary hypertension is a pulmonary arterial hypertension.
29. The methods or the pharmaceutical product according to item 28, wherein the pulmonary arterial hypertension is a WHO Group I or WHO Group III pulmonary arterial hypertension.
30. A kit comprising
   a) a first liquid composition comprising Treprostinil or a pharmaceutically acceptable salt or derivative thereof in a first concentration; and
   b) a second liquid composition comprising Treprostinil or a pharmaceutically acceptable salt or derivative thereof in a second concentration; and optionally
   c) a third liquid composition comprising Treprostinil or a pharmaceutically acceptable salt or derivative thereof in a third concentration;
   wherein the second concentration of Treprostinil or a pharmaceutically acceptable salt or derivative thereof in the second liquid composition is higher than the first concentration of Treprostinil or a pharmaceutically acceptable salt or derivative thereof in the first liquid composition and wherein the third concentration of Treprostinil or a pharmaceutically acceptable salt or derivative thereof in the optional third liquid composition is higher than the second concentration of Treprostinil or a pharmaceutically acceptable salt or derivative thereof in the second liquid composition.
31. The kit according to item 30, wherein the first, the second and the optional third liquid composition comprising Treprostinil or a pharmaceutically acceptable salt or derivative thereof are provided separately in a first, a second and optionally a third container.
32. The kit according to item 31, further comprising a first, a second and optionally a third soft mist inhaler, wherein each soft mist inhaler comprises one of the first, second and optionally third cartridge.
33. The kit according to item 31, further comprising a single soft mist inhaler adapted to consecutively receive the first, second and optional third cartridge.
34. The kit according to any one of items 30 to 33, further comprising instructions for use of the first, second and optional third container.

The invention further relates to the following numbered items (list of numbered items 2/2).
1. A pharmaceutical product comprising Treprostinil or a pharmaceutically acceptable salt or derivative thereof for use in the treatment of pulmonary hypertension in a subject being diagnosed with pulmonary hypertension or for use in the prevention of pulmonary hypertension in a subject being at risk of developing pulmonary hypertension wherein the treatment or prevention comprises the steps of
   i) administering to said subject a first liquid composition comprising Treprostinil or a pharmaceutically acceptable salt or derivative thereof in a first concentration in aerosolized form for a first treatment period, and
   ii) administering to said subject a second liquid composition comprising Treprostinil or a pharmaceutically acceptable salt or derivative thereof in a second concentration in aerosolized form for a second treatment period following the first treatment period;
   wherein the second concentration of Treprostinil or a pharmaceutically acceptable salt or derivative thereof in the second liquid composition is higher than the first concentration of Treprostinil or a pharmaceutically acceptable salt or derivative thereof in the first liquid composition, and wherein the first and the second liquid compositions are aerosolized and administered to the subject using a soft mist inhaler.
2. Treprostinil or a pharmaceutically acceptable salt or derivative thereof for use in a method of treatment of pulmonary hypertension in a subject being diagnosed with pulmonary hypertension or for use in a method of prevention of pulmonary hypertension in a subject being at risk of developing pulmonary hypertension, wherein the method comprises the steps of
   i) administering to said subject a first liquid composition comprising Treprostinil or a pharmaceutically acceptable salt or derivative thereof in a first concentration in aerosolized form for a first treatment period, and
   ii) administering to said subject a second liquid composition comprising Treprostinil or a pharmaceutically acceptable salt or derivative thereof in a second concentration in aerosolized form for a second treatment period following the first treatment period;

   wherein the second concentration of Treprostinil or a pharmaceutically acceptable salt or derivative thereof in the second liquid composition is higher than the first concentration of Treprostinil or a pharmaceutically acceptable salt or derivative thereof in the first liquid composition, and
   wherein the first and the second liquid compositions are aerosolized and administered to the subject using a soft mist inhaler.
3. The pharmaceutical product for use according to item 1 or Treprostinil or a pharmaceutically acceptable salt or derivative thereof for use according to item 2, wherein the treatment further comprising the step of
   iii) administering a third liquid composition comprising Treprostinil or a pharmaceutically acceptable salt or derivative thereof in a third concentration in aerosolized form for a third treatment period following the second treatment period;
   wherein the third concentration of Treprostinil or a pharmaceutically acceptable salt or derivative thereof in the third liquid composition is higher than the second concentration of Treprostinil or a pharmaceutically acceptable salt or derivative thereof in the second liquid composition, and wherein the third liquid composition is aerosolized and administered to the subject using a soft mist inhaler.
4. The pharmaceutical product or Treprostinil or a pharmaceutically acceptable salt or derivative thereof for use according to any one of the preceding items, wherein the concentration of Treprostinil or a pharmaceutically acceptable salt or derivative thereof in the first liquid composition is selected within a range of from about 1 mg/mL to about 3 mg/mL.
5. The pharmaceutical product or Treprostinil or a pharmaceutically acceptable salt or derivative thereof for use according to any one of the preceding items, wherein the concentration of Treprostinil or a pharmaceutically acceptable salt or derivative thereof in the second liquid composition is selected within a range of from about 2 mg/mL to about 4 mg/mL.
6. The pharmaceutical product or Treprostinil or a pharmaceutically acceptable salt or derivative thereof for use according to any one of the preceding items, wherein the concentration of Treprostinil or a pharmaceutically acceptable salt or derivative thereof in the third liquid composition is selected within a range of from about 3 mg/mL to about 6 mg/mL.
7. The pharmaceutical product or Treprostinil or a pharmaceutically acceptable salt or derivative thereof for use according to any one of the preceding items, wherein the first, the second and the optional third liquid composition each is an aqueous solution comprising an aqueous solvent or mixture of solvents.
8. The pharmaceutical product or Treprostinil or a pharmaceutically acceptable salt or derivative thereof for use according to any one of the preceding items, wherein the first, the second and the optional third liquid composition further comprises one or more pharmaceutically acceptable excipients.
9. The pharmaceutical product or Treprostinil or a pharmaceutically acceptable salt or derivative thereof for use according to any one of the preceding items, wherein the duration of the first treatment period is selected within the range of from about 1 day to about 15 days.
10. The pharmaceutical product or Treprostinil or a pharmaceutically acceptable salt or derivative thereof for use according to any one of the preceding items, wherein the duration of the second treatment period is selected within the range of from about 1 day to about 15 days.
11. The pharmaceutical product or Treprostinil or a pharmaceutically acceptable salt or derivative thereof for use according to any one of the preceding items, wherein the amount of Treprostinil or a pharmaceutically acceptable salt or derivative thereof administered in a single event during the first treatment period is selected with the range of from about 10 µg to about 25 µg, preferably of from about 15 µg to about 20 µg.
12. The pharmaceutical product or Treprostinil or a pharmaceutically acceptable salt or derivative thereof for use according to any one of the preceding items, wherein the amount of Treprostinil or a pharmaceutically acceptable salt or derivative thereof administered in a single event during the second treatment period is selected with the range of from about 25 µg to about 50 µg, preferably of from about 30 µg to about 40 µg.
13. The pharmaceutical product or Treprostinil or a pharmaceutically acceptable salt or derivative thereof for use according to any one of the preceding items, wherein the amount of Treprostinil or a pharmaceutically acceptable salt or derivative thereof administered in a single event during the third treatment period is selected with the range of from about 40 µg to about 70 µg, preferably of from about 45 µg to about 60 µg.
14. The pharmaceutical product or Treprostinil or a pharmaceutically acceptable salt or derivative thereof for use according to any one of the preceding items, wherein the amount of Treprostinil or a pharmaceutically acceptable salt or derivative thereof to be administered in a single event during the first, second and optional third treatment period is administered in one breath each upon a single activation of the soft mist inhaler upon which a single metered dose of Treprostinil or a pharmaceutically acceptable salt or derivative is dispensed.
15. The pharmaceutical product or Treprostinil or a pharmaceutically acceptable salt or derivative thereof for use according to any one of the preceding items, wherein Treprostinil or a pharmaceutically acceptable salt or derivative thereof is administered to the subject during the first treatment period in a daily dose selected within a range of from about 40 µg to about 100 µg.
16. The pharmaceutical product or Treprostinil or a pharmaceutically acceptable salt or derivative thereof for use according to any one of the preceding items, wherein Treprostinil or a pharmaceutically acceptable salt or derivative thereof is administered to the subject during the second treatment period in a daily dose selected within a range of from about 100 µg to about 200 µg.
17. The pharmaceutical product or Treprostinil or a pharmaceutically acceptable salt or derivative thereof for use according to any one of the preceding items, wherein Treprostinil or a pharmaceutically acceptable salt or derivative thereof is administered to the subject during the third treatment period in a daily dose selected within a range of from about 160 µg to about 280 µg.
18. The pharmaceutical product or Treprostinil or a pharmaceutically acceptable salt or derivative thereof for use according to any one of the preceding items, wherein Treprostinil or a pharmaceutically acceptable salt or derivative thereof in the first, the second and the optional third liquid composition is Treprostinil sodium.
19. The pharmaceutical product or Treprostinil or a pharmaceutically acceptable salt or derivative thereof for use according to any one of the preceding items, wherein the soft mist inhaler is adapted to generate an aerosol or plume of aerosol of the first, second and optional third liquid composition having an average velocity of 1.0 to 2.5 m/s, preferably of 1.3 to 2.1 m/s.
20. The pharmaceutical product or Treprostinil or a pharmaceutically acceptable salt or derivative thereof for use according to any one of the preceding items, wherein the soft mist inhaler is adapted to hold the first, second and the optional third liquid composition comprising Treprostinil or a pharmaceutically acceptable salt or derivative thereof at atmospheric pressure.
21. The pharmaceutical product or Treprostinil or a pharmaceutically acceptable salt or derivative thereof for use according to any one of the preceding items, wherein the soft mist inhaler is adapted to aerosolize the first, second and optional third liquid composition comprising Treprostinil or a pharmaceutically acceptable salt or derivative thereof by an entirely mechanical mechanism, preferably by a mechanism that does not comprise electronic means.
22. The pharmaceutical product or Treprostinil or a pharmaceutically acceptable salt or derivative thereof for use according to any one of the preceding items, wherein the soft mist inhaler comprises a nozzle for the aerosolization of the first, second and optional third liquid composition comprising Treprostinil or a pharmaceutically acceptable salt or derivative thereof.
23. The pharmaceutical product or Treprostinil or a pharmaceutically acceptable salt or derivative thereof for use according to any one of the preceding items, wherein the soft mist inhaler comprises a pumping unit adapted to generate a working pressure of the first second and optional third liquid composition comprising Treprostinil or a pharmaceutically acceptable salt or derivative thereof within the range of from about 50 to about 350 bar.
24. The pharmaceutical product or Treprostinil or a pharmaceutically acceptable salt or derivative thereof for use according to any one of the preceding items, wherein the soft mist inhaler is an impingement-type soft mist inhaler having at least one nozzle with at least two ejection channels adapted to generate at least two jets of the first, second and optional third liquid composition comprising Treprostinil or a pharmaceutically acceptable salt or derivative thereof that intersect to generate an aerosol.
25. The pharmaceutical product or Treprostinil or a pharmaceutically acceptable salt or derivative thereof for use according to any one of the preceding items, wherein the soft mist inhaler is a hand-held inhalation device for delivering a nebulised medically active aerosol for inhalation therapy, comprising
   (a) a housing having a user-facing side;
   (b) an impingement-type nozzle for generating the nebulised aerosol by collision of at least two liquid jets, the nozzle being firmly affixed to the user-facing side of the housing such as to be immobile relative to the housing;
   (c) a fluid reservoir arranged within the housing; and
   (d) a pumping unit arranged within the housing, the pumping unit having
      - an upstream end that is fluidically connected to the fluid reservoir;
      - a downstream end that is fluidically connected to the nozzle;

   wherein the pumping unit is adapted for pumping fluid from the fluid reservoir to the nozzle;
   wherein the pumping unit further comprises
      (i) a riser pipe having an upstream end, wherein the riser pipe is
         - adapted to function as a piston in the pumping unit, and
         - firmly affixed to the user-facing side of the housing such as to be immobile relative to the housing; and
      (ii) a hollow cylinder located upstream of the riser pipe, wherein the upstream end of the riser pipe is inserted in the cylinder such that the cylinder is longitudinally movable on the riser pipe;
      (iii) a lockable means for storing potential energy when locked and for releasing the stored energy when unlocked, the means being arranged outside of, and mechanically coupled to, the cylinder such that unlocking the means results in a propulsive longitudinal movement of the cylinder towards the downstream end of the pumping unit.
26. A method for the inhalative administration of Treprostinil or a pharmaceutically acceptable salt or derivative thereof to a subject in need of such administration, the method comprising the steps of
   i) administering to said subject a first liquid composition comprising Treprostinil or a pharmaceutically acceptable salt or derivative thereof in a first concentration in aerosolized form for a first treatment period, and
   ii) administering to said subject a second liquid composition comprising Treprostinil or a pharmaceutically acceptable salt or derivative thereof in a second concentration in aerosolized form for a second treatment period following the first treatment period;

   wherein the second concentration of Treprostinil or a pharmaceutically acceptable salt or derivative thereof in the second liquid composition is higher than the first concentration of Treprostinil or a pharmaceutically acceptable salt or derivative thereof in the first liquid composition, and
   wherein the first and the second liquid compositions are aerosolized and administered to the subject using a soft mist inhaler.
27. The method according to item 26, wherein the subject is diagnosed with pulmonary hypertension or is at risk of developing pulmonary hypertension.
28. A method of treatment of pulmonary hypertension in a subject suffering from pulmonary hypertension or for preventing pulmonary hypertension in a subject being at risk of developing pulmonary hypertension, the method comprising the method for the inhalative administration of Treprostinil or a pharmaceutically acceptable salt or derivative thereof according to item 26 or 27.
29. The methods or the pharmaceutical product according any one of the preceding items, wherein the pulmonary hypertension is a pulmonary arterial hypertension.
30. The methods or the pharmaceutical product according to item 29, wherein the pulmonary arterial hypertension is a WHO Group I or WHO Group III pulmonary arterial hypertension.
31. A kit comprising
   a) a first liquid composition comprising Treprostinil or a pharmaceutically acceptable salt or derivative thereof in a first concentration; and
   b) a second liquid composition comprising Treprostinil or a pharmaceutically acceptable salt or derivative thereof in a second concentration; and optionally
   c) a third liquid composition comprising Treprostinil or a pharmaceutically acceptable salt or derivative thereof in a third concentration;
   wherein the second concentration of Treprostinil or a pharmaceutically acceptable salt or derivative thereof in the second liquid composition is higher than the first concentration of Treprostinil or a pharmaceutically acceptable salt or derivative thereof in the first liquid composition and wherein the third concentration of Treprostinil or a pharmaceutically acceptable salt or derivative thereof in the optional third liquid composition is higher than the second concentration of Treprostinil or a pharmaceutically acceptable salt or derivative thereof in the second liquid composition.
32. The kit according to item 31, wherein the first, the second and the optional third liquid composition comprising Treprostinil or a pharmaceutically acceptable salt or derivative thereof are provided separately in a first, a second and an optional third container.
33. The kit according to item 31or 32, further comprising a first, a second and optionally a third soft mist inhaler, wherein each soft mist inhaler comprises one of the first, second and optionally third liquid composition.
34. The kit according to item 32, further comprising a single soft mist inhaler adapted to consecutively receive the first, second and optional third container.
35. The kit according to any one of items 31 to 34, further comprising instructions for use of the first, second and optional third container.

The following examples serve to illustrate the invention, however, said examples should not to be understood as restricting the scope of the invention.

### EXAMPLES

### Example 1:

A liquid clear solution of Treprostinil sodium in water with a concentration of 1.0 mg/mL was prepared and filled into an impingement-type soft mist inhaler having two ejection channels with a diameter of 8 µm, a dose volume of 15 µL and an operating pressure of 250 bar as further described in Example 5 below. Spraying experiments resulted in the formation of plumes of an aerosol having the characteristics as summarized in Table 1, whereas "Event duration" [s] means the time span necessary for the generation of the aerosol plume of a single activation of the soft mist inhaler, "Dv 10/50/90" [µm] means the volumetric parameters of the droplet size distribution, "V<10/<5 µm" [%] means the volume of all droplets having a diameter of below 10 µm/5µm, "GSD" [µm] means the geometric standard deviation, "St_{dev}" means the standard deviation, "N" means the number of experiments, "Stₑᵣᵣₒᵣ" means the standard error, "CI" means the confidence interval, and "RSD" means the relative standard deviation.

**Table 1: Droplet Size Distribution results obtained when nebulizing a 1.0 mg/mL water solution of Treprostinil with an impingement-type soft mist inhaler**

| | Average | St_{dev} | N | Stₑᵣᵣₒᵣ | CI 95% | RSD |
|---|---|---|---|---|---|---|
| Event duration / s | 1.76 | 0.03 | 6 | 0.01 | 0.03 | 1.69% |
| Dv10 / µm | 1.33 | 0.34 | 6 | 0.14 | 0.35 | 25.20% |
| Dv50 / µm | 5.32 | 0.37 | 6 | 0.15 | 0.39 | 6.92% |
| Dv90 / µm | 12.48 | 2.16 | 6 | 0.88 | 2.27 | 17.33% |
| V < 10µm / % | 83.63 | 4.88 | 6 | 1.99 | 5.12 | 5.84% |
| GSD / µm | 3.31 | 0.29 | 6 | 0.12 | 0.31 | 8.89% |
| V < 5 µm / % | 46.75 | 3.75 | 6 | 1.53 | 3.94 | 8.03% |

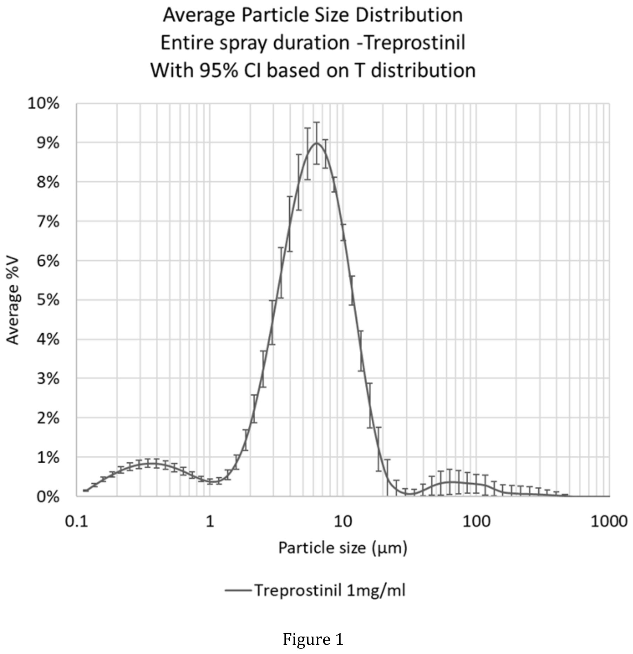

The corresponding droplet size distribution of the generated aerosol was measured with a Laser Diffraction system (Spraytec device from Malvern), and is shown in Fig. 1. As can be seen from the graph, the maximum of the droplet size distribution is below 5 µm. More specifically, 83.6 % of all droplets have a diameter of below 10 µm and 46.8 % of all droplets have a diameter of below 5 µm.

### Example 2:

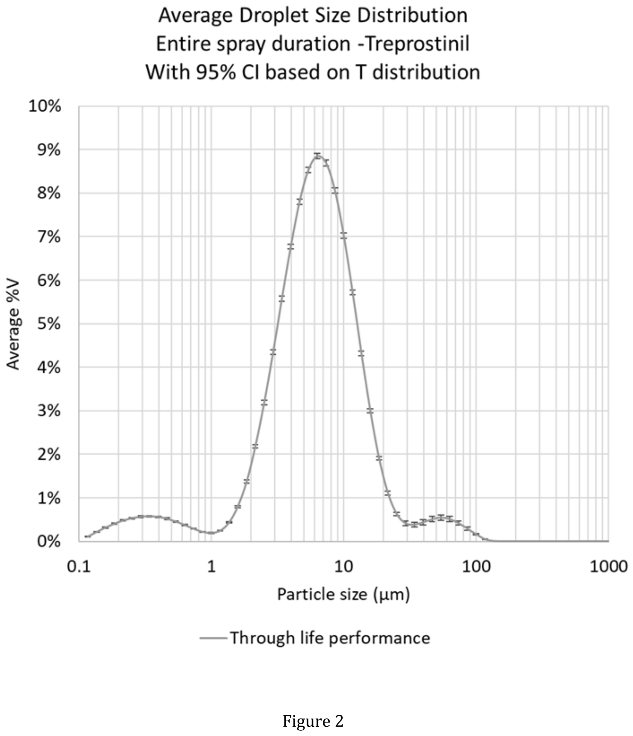

In this example the suitability for long-term performance of the chosen soft mist inhaler as described in Examples 1 and 5 for the generation of an aerosol comprising Treprostinil sodium was investigated. The device was actuated 120 times in series of 6 actuations and the event duration (timespan necessary for the generation of the aerosol plume of a single activation) as well as the particle (droplet) size distribution was determined for each series of 6 actuations. The results are summarized in Table 2 and in Figure 2, presented below:

**Table 2: Average Droplet Size Distribution results obtained with the combination of impingement-type soft mist inhaler with a 1.0 mg/mL water solution of Treprostinil**

| Parameters | Through life | |
|---|---|---|
| | Performance | |
| | Mean (n=120) | Standard deviation |
| Event duration [s] | 1.68 | 0.08 |
| Dv10 [µm] | 2.05 | 0.08 |
| Dv50 [µm] | 5.71 | 0.19 |
| Dv90 [µm] | 14.23 | 2.14 |

As a result of this long-term study it was found that the that the event duration as well as the particle size distribution remained stable after 120 actuations.

### Example 3:

A liquid clear solution of free Treprostinil in water with a concentration of 5.0 mg/mL was prepared and filled into the same impingement-type soft mist inhaler as described in Example 1. Spraying experiments resulted in the formation of plumes of an aerosol having the characteristics as summarized in Table 3.

**Table 3: Droplet Size Distribution results obtained with the combination of impingement-type soft mist inhaler with a 5.0 mg/mL water solution of Treprostinil**

| | Average | St_{dev} | N | Stₑᵣᵣₒᵣ | CI 95% | RSD |
|---|---|---|---|---|---|---|
| Event duration / s | 1.40 | 0.01 | 6 | 0.00 | 0.01 | 0.47% |
| Dv10 /µm | 2.73 | 0.01 | 6 | 0.01 | 0.02 | 0.52% |
| Dv50 / µm | 4.75 | 0.02 | 6 | 0.01 | 0.02 | 0.38% |
| Dv90 / µm | 7.99 | 0.03 | 6 | 0.01 | 0.03 | 0.35% |
| V < 10µm / % | 97.35 | 0.07 | 6 | 0.03 | 0.07 | 0.07% |
| GSD / µm | 3.87 | 0.28 | 6 | 0.12 | 0.30 | 7.34% |
| V < 5 µm / % | 54.84 | 0.34 | 6 | 0.14 | 0.36 | 0.63% |

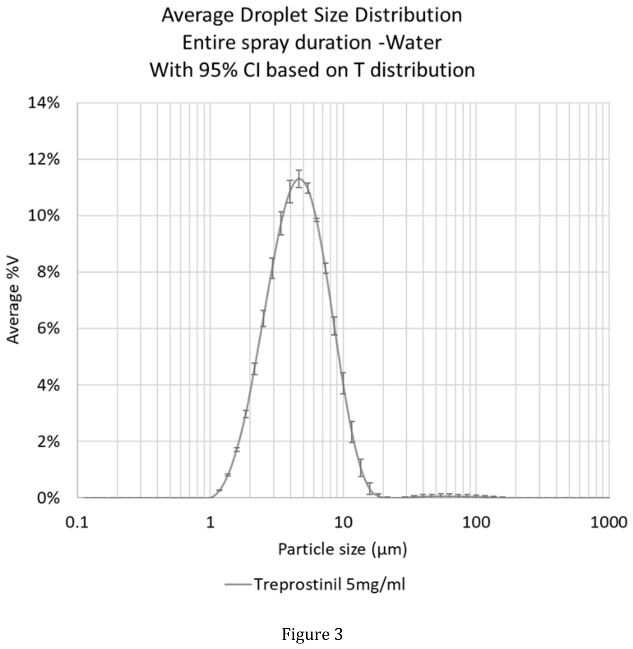

The corresponding droplet size distribution was measured with a Laser Diffraction system (Spraytec device from Malvern) and is shown in Fig. 3. As can be seen from the graph the maximum of the droplet size distribution is below 5 µm. More specifically, 97 % of all droplets have a diameter of below 10 µm and almost 55% of all droplets have a diameter of below 5 µm.

### Example 4:

A liquid clear solution of free Treprostinil in a solution containing 50% water and 50% ethanol (v/v) with a concentration of 5.0 mg/mL was prepared and filled into the same impingement-type soft mist inhaler device as described in Example 1. Spraying experiments resulted in the formation of plumes of an aerosol having the characteristics as summarized in Table 4 below.

**Table 4: Droplet Size Distribution results obtained with the combination of impingement-type soft mist inhaler with a 5.0mg/ml water/ethanol (50%/50% v/v) solution of Treprostinil**

| | Average | St_{dev} | N | Stₑᵣᵣₒᵣ | CI 95% | RSD |
|---|---|---|---|---|---|---|
| Event duration / s | 2.95 | 0.06 | 6 | 0.03 | 0.07 | 2.16% |
| Dv10 /µm | 2.40 | 0.06 | 6 | 0.02 | 0.06 | 2.34% |
| Dv50 / µm | 5.88 | 0.41 | 6 | 0.17 | 0.43 | 6.95% |
| Dv90 / µm | 22.38 | 3.45 | 6 | 1.41 | 3.62 | 15.43% |
| V < 10µm / % | 74.13 | 4.00 | 6 | 1.63 | 4.20 | 5.40% |
| GSD / µm | 3.31 | 0.16 | 6 | 0.07 | 0.17 | 4.91% |
| V < 5 µm / % | 41.75 | 2.90 | 6 | 1.18 | 3.04 | 6.94% |

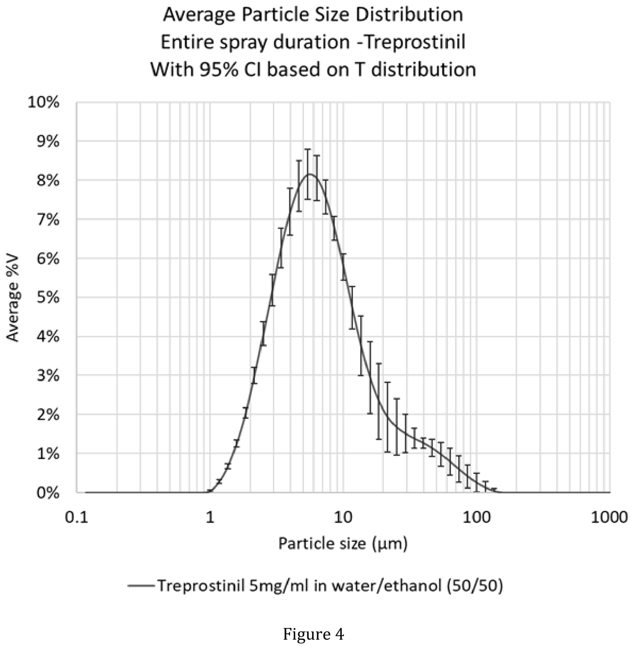

The corresponding droplet size distribution was measured with a Laser Diffraction system (Spraytec device from Malvern) and is shown in Fig. 4. As can be seen, 74.1 % of all droplets have a diameter of below 10 µm and 41.8% of all droplets have a diameter of below 5 µm.

### Example 5:

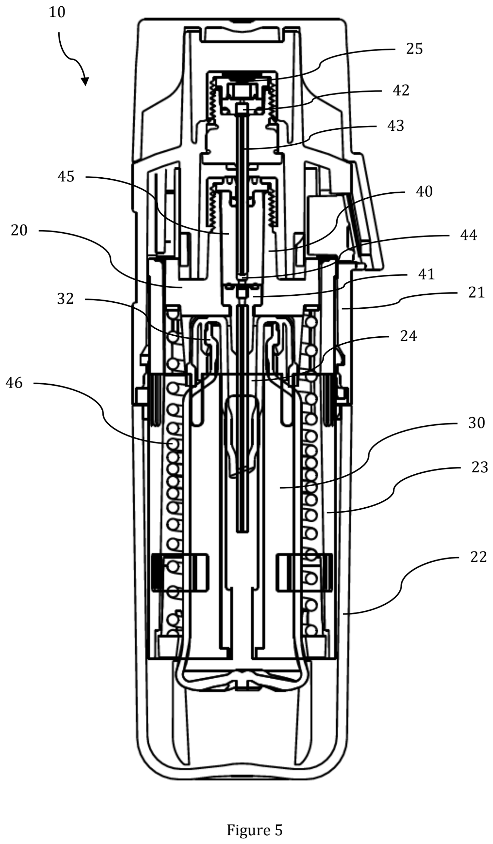

Fig. 5 shows a cross-sectional view of a soft mist inhaler (10) suitable for the implementation in the methods, compositions for use as well as the kits according to the present invention comprising an inhalation device (20) and an exchangeable reservoir in the form of a cartridge or container (30) inserted into the inhalation device. The inhalation device (20) has a housing (21) with a lower part (22) that can be detached from the inhalation device (20) and removed to open the housing (21) and allow access to a receiving unit (23) in which the exchangeable container (30) can be inserted. The receiving unit (23) further has a connection unit (24) adapted to releasably and fluidically connect to a connection port (32) of the exchangeable container (30).

The inhalation device (20) further has a nozzle (25) located at the downstream end of the inhalation device for aerosolization of the first, second or third liquid composition, respectively, provided in the exchangeable container (30). The inhalation device further has a pumping unit (40) which is arranged within the housing (21). The pumping unit is fluidically connected to the container (30) (via the connection unit (24) of the receiving unit (23)) and to the nozzle (25) and is adapted to pump the first, second or third liquid composition, respectively, in a downstream direction from the container (30) to the nozzle (25).

The pumping unit (40) has an upstream end (41) that is fluidically connected to the exchangeable container (30); a downstream end (42) that is fluidically connected to the nozzle (25); wherein the pumping unit (40) further comprises (i) a riser pipe (43) having an upstream end (44), wherein the riser pipe (43) is adapted to function as a piston in the pumping unit (40), and wherein the riser pipe (43) is firmly affixed to the user-facing (downstream) side of the housing (21) such as to be immobile relative to the housing (21); and (ii) a hollow cylinder (45) located upstream of the riser pipe (43), wherein the upstream end of the riser pipe (44) is inserted in the cylinder (45) such that the cylinder (45) is longitudinally movable on the riser pipe (43).

As also shown in Fig. 5, the pumping unit (40) comprises (iii) a lockable means for storing potential energy (46) when locked and for releasing the stored energy when unlocked, the means (46) being arranged outside of, and mechanically coupled to, the cylinder (45) such that unlocking the means (46) results in a propulsive longitudinal movement of the cylinder (45) towards the downstream end of the pumping unit (42).

## Claims

1. Treprostinil or a pharmaceutically acceptable salt thereof for use in a method of treatment of pulmonary hypertension in a subject being diagnosed with pulmonary hypertension or for use in a method of prevention of pulmonary hypertension in a subject being at risk of developing pulmonary hypertension, wherein the method comprises the steps of
i) administering to said subject a first liquid composition comprising Treprostinil or a pharmaceutically acceptable salt thereof in a first concentration, within a range of from 1 mg/mL to 3 mg/mL, in aerosolized form for a first treatment period,
ii) administering to said subject a second liquid composition comprising Treprostinil or a pharmaceutically acceptable salt thereof in a second concentration, within a range of from 2 mg/mL to 4 mg/mL, in aerosolized form for a second treatment period following the first treatment period; and
iii) administering a third liquid composition comprising Treprostinil or a pharmaceutically acceptable salt thereof in a third concentration, within a range of from 3 mg/mL to 6 mg/mL, in aerosolized form for a third treatment period following the second treatment period;
wherein the second concentration of Treprostinil or a pharmaceutically acceptable salt thereof in the second liquid composition is higher than the first concentration of Treprostinil or a pharmaceutically acceptable salt thereof in the first liquid composition, and wherein the third concentration of Treprostinil or a pharmaceutically acceptable salt thereof in the third liquid composition is higher than the second concentration of Treprostinil or a pharmaceutically acceptable salt thereof in the second liquid composition, and
wherein the first, second and third liquid compositions are aerosolized and administered to the subject using the same soft mist inhaler.

2. Treprostinil or a pharmaceutically acceptable salt thereof for use according to claim 1, wherein the first, the second and the third liquid composition each is an aqueous solution comprising an aqueous solvent or mixture of solvents.

3. Treprostinil or a pharmaceutically acceptable salt thereof for use according to any one of the preceding claims, wherein the amount of Treprostinil or a pharmaceutically acceptable salt thereof administered in a single event during the first treatment period is selected with the range of from about 10 µg to about 25 µg, preferably of from about 15 µg to about 20 µg.

4. Treprostinil or a pharmaceutically acceptable salt thereof for use according to any one of the preceding claims, wherein the amount of Treprostinil or a pharmaceutically acceptable salt thereof administered in a single event during the second treatment period is selected with the range of from about 25 µg to about 50 µg, preferably of from about 30 µg to about 40 µg.

5. Treprostinil or a pharmaceutically acceptable salt thereof for use according to any one of the preceding claims, wherein the amount of Treprostinil or a pharmaceutically acceptable salt thereof administered in a single event during the third treatment period is selected with the range of from about 40 µg to about 70 µg, preferably of from about 45 µg to about 60 µg.

6. Treprostinil or a pharmaceutically acceptable salt thereof for use according to any one of the preceding claims, wherein the amount of Treprostinil or a pharmaceutically acceptable salt thereof to be administered in a single event during the first, second and third treatment period is administered in one breath each upon a single activation of the soft mist inhaler upon which a single metered dose of Treprostinil or a pharmaceutically acceptable salt is dispensed.

7. Treprostinil or a pharmaceutically acceptable salt thereof for use according to any one of the preceding claims, wherein the Treprostinil or a pharmaceutically acceptable salt thereof is administered to the subject during the first treatment period in a daily dose selected within a range of from about 40 µg to about 100 µg.

8. Treprostinil or a pharmaceutically acceptable salt thereof for use according to any one of the preceding claims, wherein the Treprostinil or a pharmaceutically acceptable salt thereof is administered to the subject during the second treatment period in a daily dose selected within a range of from about 100 µg to about 200 µg.

9. Treprostinil or a pharmaceutically acceptable salt thereof for use according to any one of the preceding claims, wherein the Treprostinil or a pharmaceutically acceptable salt thereof is administered to the subject during the third treatment period in a daily dose selected within a range of from about 160 µg to about 280 µg.

10. Treprostinil or a pharmaceutically acceptable salt thereof for use according to any one of the preceding claims, wherein the soft mist inhaler is adapted to generate an aerosol or plume of aerosol of the first, second and third liquid composition having an average velocity of 1.0 to 2.5 m/s, preferably of 1.3 to 2.1 m/s.

11. Treprostinil or a pharmaceutically acceptable salt thereof for use according to any one of the preceding claims, wherein the soft mist inhaler is adapted to hold the first, second and the third liquid composition comprising Treprostinil or a pharmaceutically acceptable salt thereof at atmospheric pressure.

12. Treprostinil or a pharmaceutically acceptable salt thereof for use according to any one of the preceding claims, wherein the soft mist inhaler comprises a pumping unit adapted to generate a working pressure of the first second and third liquid composition comprising Treprostinil or a pharmaceutically acceptable salt thereof within the range of from about 50 to about 350 bar.

13. Treprostinil or a pharmaceutically acceptable salt thereof for use according to any one of the preceding claims, wherein the soft mist inhaler is an impingement-type soft mist inhaler having at least one nozzle with at least two ejection channels adapted to generate at least two jets of the first, second and third liquid composition comprising Treprostinil or a pharmaceutically acceptable salt thereof that intersect to generate an aerosol.

14. Treprostinil or a pharmaceutically acceptable salt thereof for use according to any one of the preceding claims, wherein the soft mist inhaler is a hand-held inhalation device for delivering a nebulised medically active aerosol for inhalation therapy, comprising
(a) a housing having a user-facing side;
(b) an impingement-type nozzle for generating the nebulised aerosol by collision of at least two liquid jets, the nozzle being firmly affixed to the user-facing side of the housing such as to be immobile relative to the housing;
(c) a fluid reservoir arranged within the housing; and
(d) a pumping unit arranged within the housing, the pumping unit having
- an upstream end that is fluidically connected to the fluid reservoir;
- a downstream end that is fluidically connected to the nozzle;
wherein the pumping unit is adapted for pumping fluid from the fluid reservoir to the nozzle;
wherein the pumping unit further comprises
(i) a riser pipe having an upstream end, wherein the riser pipe is
- adapted to function as a piston in the pumping unit, and
- firmly affixed to the user-facing side of the housing such as to be immobile relative to the housing; and
(ii) a hollow cylinder located upstream of the riser pipe, wherein the upstream end of the riser pipe is inserted in the cylinder such that the cylinder is longitudinally movable on the riser pipe;
(iii) a lockable means for storing potential energy when locked and for releasing the stored energy when unlocked, the means being arranged outside of, and mechanically coupled to, the cylinder such that unlocking the means results in a propulsive longitudinal movement of the cylinder towards the downstream end of the pumping unit.

15. A kit comprising:
a) a first liquid composition comprising Treprostinil or a pharmaceutically acceptable salt thereof in a first concentration within a range of from 1 mg/mL to 3 mg/mL; and
b) a second liquid composition comprising Treprostinil or a pharmaceutically acceptable salt thereof in a second concentration within a range of from 2 mg/mL to 4 mg/mL; and
c) a third liquid composition comprising Treprostinil or a pharmaceutically acceptable salt thereof in a third concentration within a range of from 3 mg/mL to 6 mg/mL;
wherein the second concentration of Treprostinil or a pharmaceutically acceptable salt thereof in the second liquid composition is higher than the first concentration of Treprostinil or a pharmaceutically acceptable salt thereof in the first liquid composition and wherein the third concentration of Treprostinil or a pharmaceutically acceptable salt thereof in the third liquid composition is higher than the second concentration of Treprostinil or a pharmaceutically acceptable salt thereof in the second liquid composition;
wherein the first, the second and the third liquid composition are provided separately in a first, a second and a third container;
and
wherein the kit further comprises a single soft mist inhaler adapted to consecutively receive the first, second and third container.
